# EUROPEAN PATENT SPECIFICATION

(11) **EP 4 291 901 B1**
(45) Date of publication and mention of the grant of the patent: **07.05.2025**
(21) Application number: 22708505.7
(22) Date of filing: 11.02.2022
(51) Int. Cl.: G01N 33/574, G01N 33/50

(54) **USE OF MARKERS FOUND ON LYMPH NODE METASTATIC SAMPLES FOR THE PROGNOSIS OF BREAST CANCER**
VERWENDUNG VON AUF METASTASTISCHEN PROBEN VON LYMPHKNOTEN GEFUNDENEN MARKERN FÜR DIE PROGNOSE VON BRUSTKREBS
UTILISATION DE MARQUEURS TROUVÉS SUR DES ÉCHANTILLONS MÉTASTATIQUES DE GANGLIONS LYMPHATIQUES POUR LE PRONOSTIC DU CANCER DU SEIN

(30) Priority: 11.02.2021 EP 21156709
(43) Date of publication of application: 20.12.2023
(73) Proprietor: Universität Zürich, 8006 Zürich (CH)
(72) Inventor: FISCHER, Jana, 8057 Zürich (CH); JACKSON, Hartland, Toronto, Ontario, M5G 0A3 (CA); BODENMILLER, Bernd, 8057 Zürich (CH)
(74) Representative: Schulz Junghans Patentanwälte PartGmbB
(86) International application number: PCT/EP2022/053429
(87) International publication number: WO 2022/171831

(56) References cited:
- ANNA-KARIN FALCK ET AL: "Does Analysis of Biomarkers in Tumor Cells in Lymph Node Metastases Give Additional Prognostic Information in Primary Breast Cancer?", WORLD JOURNAL OF SURGERY ; OFFICIAL JOURNAL OF THE INTERNATIONALSOCIETY OF SURGERY/SOCIÉTÉ INTERNATIONALE DE CHIRURGIE, SPRINGER-VERLAG, NE, vol. 34, no. 7, 6 March 2010 (2010-03-06), pages 1434 - 1441, XP019842663, ISSN: 1432-2323
- CALVO JULIA ET AL: "Infrequent Loss of Luminal Differentiation in Ductal Breast Cancer Metastasis", PLOS ONE, vol. 8, no. 10, 21 October 2013 (2013-10-21), pages e78097, XP055921934, Retrieved from the Internet <URL:https://journals.plos.org/plosone/article/file?id=10.1371/journal.pone.0078097&type=printable> DOI: 10.1371/journal.pone.0078097
- SHIELD PAUL W ET AL: "Gata3 Immunohistochemical Staining is A Useful Marker for Metastatic Breast Carcinoma in Fine Needle Aspiration Specimens - PMC", JOURNAL OF CYTOLOGY, 22 March 2018 (2018-03-22), pages 1 - 8, XP055921979, Retrieved from the Internet <URL:https://www.ncbi.nlm.nih.gov/pmc/articles/PMC5885610/?report=printable> [retrieved on 20220517]
- VODUC DAVID ET AL: "GATA-3 Expression in Breast Cancer Has a Strong Association with Estrogen Receptor but Lacks Independent Prognostic Value", CANCER EPIDEMIOLOGY, BIOMARKERS & PREVENTION, vol. 17, no. 2, 1 February 2008 (2008-02-01), pages 365 - 373, XP055921982, ISSN: 1055-9965, Retrieved from the Internet <URL:https://watermark.silverchair.com/365.pdf?token=AQECAHi208BE49Ooan9kkhW_Ercy7Dm3ZL_9Cf3qfKAc485ysgAAAswwggLIBgkqhkiG9w0BBwagggK5MIICtQIBADCCAq4GCSqGSIb3DQEHATAeBglghkgBZQMEAS4wEQQM4fmmnsLodmmBgd5VAgEQgIICf2g032h_hvHEiuEF8u3tlVKfDxyrQFweW-WiyiyunE_mWPM4RLofrCPc-WtLM1AmIaZqnktVm_PecrsB1wKTj3VMeYeHX9xg> DOI: 10.1158/1055-9965.EPI-06-1090
- CARDOSO F. ET AL: "Evaluation of HER2, p53, bcl-2, topoisomerase II-[alpha], heat shock proteins 27 and 70 in primary breast cancer and metastatic ipsilateral axillary lymph nodes", ANNALS OF ONCOLOGY, vol. 12, no. 5, 1 May 2001 (2001-05-01), NL, pages 615 - 620, XP055922430, ISSN: 0923-7534, DOI: 10.1023/A:1011182524684
- JACQUEMIER JOCELYNE ET AL: "Association of GATA3, P53, Ki67 status and vascular peritumoral invasion are strongly prognostic in luminal breast cancer", BREAST CANCER RESEARCH, CURRENT MEDICINE GROUP LTD, GB, vol. 11, no. 2, 30 April 2009 (2009-04-30), pages R23, XP021053459, ISSN: 1465-5411, DOI: 10.1186/BCR2249
- TAKAYUKI KOBAYASHI ET AL: "A simple immunohistochemical panel comprising 2 conventional markers, Ki67 and p53, is a powerful tool for predicting patient outcome in luminal-type breast cancer", BMC CLINICAL PATHOLOGY, BIOMED CENTRAL, LONDON, GB, vol. 13, no. 1, 6 February 2013 (2013-02-06), pages 5, XP021139352, ISSN: 1472-6890, DOI: 10.1186/1472-6890-13-5

## Description

The present invention relates to a method of predicting the prognosis of a breast cancer patient by measuring the expression level of p53 and GATA3 in a lymph node metastasis sample.

### Background of the Invention

Molecular classification followed by targeted treatment has significantly improved prognosis of patients with breast cancer, yet one-third of breast cancer patients eventually develop distant metastases and succumb to disease. Although mortality is largely caused by metastases, the histologic and genetic analysis is performed on the primary tumour in current clinical care. Little is known about how disseminating cell phenotypes relate to the diverse cell types and microenvironments of the primary tumour or how these phenotypes relate to disease outcome.

The spread of tumour cells to the sentinel LNs is an important prognostic factor in breast cancer diagnosis and influences treatment decisions. Clinical classification of LN status, (nodal status) does not provide information about the biology of LN metastases, in contrast to measures such as tumour grade or molecular subtype, which are used to stratify primary tumours and also guide clinical decisions. To target disseminated tumour cells in the lymphatic system, node-positive breast cancer patients typically receive adjuvant chemotherapy. However, tumour grade and molecular subtype markers (ER, PR, HER2, and Ki-67) are not necessarily consistent through tumour progression or between primary and metastatic sites (Linstrom, L. S. 2012, J. Clin. Oncol. 30:2601). Further, some patients with high nodal status survive long term, whereas some node-negative patients do not, indicating that the amount of tumour cells in the LNs does not fully capture patient prognosis. Single-cell studies have shown substantial intra- and inter-tumour heterogeneity in primary tumours, which are associated with tumour type and distinct patient outcomes, but which single-cell phenotypes disseminate is unknown.

Falck et al. (World J. Surgery 2010; 34(7): 1434-1441) examines the distribution of ER, PR, HER2, and Ki67 in matched primary tumour and lymph node metastases in a cohort of breast cancer patients treated with tamoxifen.

Clavo et al. (PLOS ONE 2013; 8(10): e78097) examines the maintenance or loss of luminal lineage differentiation markers including GATA3 during metastases of primary ductal breast cancers with a luminal phenotype.

Shield et al. (J. Cytology 2018; 1-8) suggests GATA3 as a putative marker for metastatic breast carcinoma in fine needle aspirations, discriminating it from other non-glandular malignancies.

Voduc et al. (Cancer Epidemiology, biomarkers & prevention 2008; 17(2): 365-373) examines the association of molecular markers via immunohistochemistry in primary tumours, suggesting GATA3 associates with ER expression but lacks prognostic value for patient outcomes or treatment response in multivariate models.

Cardoso et al. (Annals Oncology 2001; 12(5): 615-620) evaluates the heterogeneity of molecule markers such as HER2, and p53 between primary breast cancer and metastatic lymph node samples, observing no markers are 100% concordant between sites in the study population.

Jaquemier et al. (Breast Cancer Res. 2009; 11(2): R23) examines the expression of molecular markers such as Ki67, p53 and GATA3 in primary tumour samples, together with morphologic classification such as the presence vascular peritumoral invasion. The study follows hormone therapy outcome in ER+ breast carcinoma patients.

Takayuki et al. (BMC Clin. Path. 2013; 13(1): 5) evaluates the prognostic value of including antibodies targeting Ki67 and p53 along with hormone receptors, in immunohistochemical panels for characterisation of patients with HR+ luminal-type breast cancer.

Based on the above-mentioned state of the art, the objective of the present invention is to provide means and methods to predict breast cancer patient outcome using biomarkers present in LN metastases. This objective is attained by the subject-matter of the independent claims of the present specification, with further advantageous embodiments described in the dependent claims, examples, figures and general description of this specification.

### Summary of the Invention

A first aspect of the invention provides a method to predict breast cancer patient prognosis, the method comprising the following steps:
a. contacting a lymph node metastasis sample obtained from the patient with a first molecular probe specific for GATA 3 conjugated to a detectable label, and a second molecular probe, conjugated to a detectable label, and specific for p53;
b. determining an expression level of GATA3 and an expression level of p53;
c. assigning to the patient a good prognosis if the patient sample is characterised by a GATA3 expression level greater than or equal to (≥) a GATA3 threshold, and/or a p53 expression level below (<) a p53 threshold, or
assigning to the patient a poor prognosis if the patient sample is characterised by a p53 expression level ≥ the p53 threshold, and/or a GATA3 expression level < the GATA3 threshold.

According to the invention, the biomarker expression level is determined in one of the following ways:
at the level of the whole sample, or a portion of the sample comprising a plurality of cells, wherein
   - the GATA3 threshold is 100 times ≥ a negative control expression level; and
   - the p53 is threshold is ≥ 8 times a negative control expression level;
and wherein the negative control expression level is the biomarker expression level determined for a negative control sample;
   or
at the level of a single cell for each of a plurality of individual cells present in the sample, wherein
   - the GATA3 threshold is ≥ 20% GATA3 high cells; and
   - the p53 threshold is ≥ 6% p53 high cells,
and wherein a cell is classified as high for biomarker expression if the biomarker expression level of the cell is ≥ 10 times the biomarker expression level of a cell in a negative control sample.

In certain embodiments of the method according to the first aspect of the invention, good prognosis indicates at least about 60% probability of survival at 300 months, and/or poor prognosis indicates less than about 50% probability of survival at 100 months.

In certain embodiments of the method according to the first aspect of the invention, good prognosis indicates about 60% probability of survival at 300 months, and/or poor prognosis indicates about 10% probability of survival at 100 months.

In certain embodiments of the method according to the first aspect of the invention, the molecular probe is an antibody, antibody-like molecule and/or nucleic acid probe, particularly wherein the molecular probe is an antibody.

In certain embodiments of the method according to the first aspect of the invention, the method of obtaining information about the expression level of the biomarkers GATA3 and/or p53 comprises obtaining an image of the ex vivo lymph node metastasis sample.

In certain embodiments of the method according to the first aspect of the invention, the sample is a monolayer of adherent ex vivo lymph node metastasis cells or ex vivo lymph node metastasis cells otherwise immobilised on a solid surface.

In certain embodiments of the method according to the first aspect of the invention, the sample is a tissue section of an ex vivo lymph node metastasis sample.

In certain embodiments of the method according to the first aspect of the invention, the method of obtaining information about the expression level of the biomarkers GATA3 and/or p53 is
a. imaging mass cytometry at a subcellular resolution, particularly at a resolution of ≤5µm, or even ≤1µm, or
b. immunohistochemistry.

A second aspect of the invention is the use of a kit comprising a molecular probe specific for biomarkers p53, and a molecular probe specific for GATA3, and each molecular probe bearing a detectable label for carrying out the method according to the first aspect of the invention.

A third aspect of the invention is a system for carrying out the method according to the first aspect of the invention, wherein the system comprises a molecular probe bearing a detectable label with specificity for p53, and a molecular probe bearing a detectable label with specificity for GATA3, and a device for analysing the amount of detectable label in a lymph node metastasis sample

### Terms and definitions

For purposes of interpreting this specification, the following definitions will apply and whenever appropriate, terms used in the singular will also include the plural and vice versa.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *p53* in the context of the present specification relates to the tumour suppressor protein cellular tumour antigen p53 (Uniprot P04637), encoded by the gene *TP53,* also known as phosphoprotein p53.

The term *GATA3* in the context of the present specification relates to Trans-acting T-cell specific transcription factor GATA-3 (Uniprot P23771), encoded by the *GATA3* gene.

The term *patient* according to the invention refers to a subject who has been diagnosed with breast cancer tumour, in other words a malignant neoplasm originating in breast tissue, specifically where the tumour has spread to lymph node (LN) tissue.

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The term *expression level* as used herein refers to a quantitative measure of gene expression, at either the mRNA or protein level. If the word "expression" is used herein in the context of "gene expression" or "expression of a marker or biomolecule" and no further qualification of "expression" is mentioned, this implies "positive expression". In the present specification, the term *high,* or *positive* when used in the context of expression of a biomarker unless specified otherwise, refers to expression level of biomarker relative to a negative control. The negative control is used to identify a background signal threshold level the label's signal when bound to a structure (for example, a cell, or a tissue lysate, or mRNA preparation) for a biomarker, and cells, or samples providing a signal above this background level in the patient sample are classified as *high* or *positive* for the biomarker. Expression level may be measured either at the level of a bulk analysis of sample, or at the level of a single cell, and optional thresholds to identify positive or high expression in these formats are provided herein.

The term *negative control* as used herein refers to a sample processed alongside the patient sample, which does not express the biomarkers, for example, a LN or tumour sample previously determined to lack p53 and/or GATA3 expression, or a healthy tissue. Suitable negative controls according to the invention include molecular probe controls, wherein a portion of the patient sample is assayed lacking the molecular probes for p53 and/or GATA3, or where the molecular probe has been exchanged for an isotype control. In particular embodiments, the biomarker expression level in the sample is compared to a healthy tissue negative control sample, either from the patient, or more particularly, from a healthy subject. A healthy liver sample to be analysed in a tissue section, or microarray such as that used in the examples as can be obtained commercially (Novusbio).

The term *molecular probe* in the context of the present specification relates to a specific ligand sensitive to expression of an antigen, particularly p53 and GATA3, at the level of mRNA, or protein expression. In embodiments relating to biomarker assessment at the protein level, molecular probes of particular use include an antibody, antibody fragment, an antibody-like molecule or aptamer, more particularly an antibody or antibody fragment, that can bind to a target molecule with a dissociation constant of ≤10⁻⁷ mol/l, particularly ≤10⁻⁸ mol/l. In other embodiments relating to biomarker mRNA expression level measurements, molecular probes of particular use are primers, complimentary nucleic acids probes specific for p53 or GATA3 mRNA, suitable for quantitative measurements of the amount of mRNA, or cDNA copies of the p53 or GATA3 gene present in sample the using polymerase chain reaction (PCR), or in situ hybridisation assays. The molecular probe according to the invention comprises a detectable marker such as a particle, bead, dye or enzyme, and must be paired with an assay sensitive to difference in the amount of molecular probe on a sample, and a control negative sample.

In the context of the present specification, the term *antibody* refers to whole antibodies including but not limited to immunoglobulin type G (IgG), type A (IgA), type D (IgD), type E (IgE) or type M (IgM), any antigen binding fragment or single chains thereof and related or derived constructs. A whole antibody is a glycoprotein comprising at least two heavy (H) chains and two light (L) chains inter-connected by disulfide bonds. Similarly, the term encompasses a so-called nanobody or single domain antibody, an antibody fragment consisting of a single monomeric variable antibody domain, or a multimeric antibody fragment molecule, or single chain variable fragment, fusion proteins of two or more variable regions connected with linker peptides.

The term *antibody-like molecule* in the context of the present specification refers to a molecule capable of specific binding to another molecule or target with high affinity / a Kd ≤ 10E-8 mol/l. An antibody-like molecule binds to its target similarly to the specific binding of an antibody. The term *antibody-like molecule* encompasses a repeat protein, such as a designed ankyrin repeat protein (Molecular Partners, Zürich), an engineered antibody mimetic proteins exhibiting highly specific and high-affinity target protein binding (US2012142611, US2016250341, US2016075767 and US2015368302). The term antibody-like molecule further encompasses, but is not limited to, a polypeptide derived from armadillo repeat proteins, a polypeptide derived from leucine-rich repeat proteins and a polypeptide derived from tetratricopeptide repeat proteins.

The term *antibody-like molecule* further encompasses a specifically binding polypeptide derived from a protein A domain, a fibronectin domain FN3, a consensus fibronectin domain, a lipocalin (see Skerra, Biochim. Biophys. Acta 2000, 1482(1-2):337-50), a polypeptide derived from a Zinc finger protein (see Kwan et al. Structure 2003, 11(7):803-813), a Src homology domain 2 (SH2) or Src homology domain 3 (SH3), a PDZ domain, a gamma-crystallin, ubiquitin, a cysteine knot polypeptide or a knottin, cystatin, Sac7d, a triple helix coiled coil (also known as alphabodies), a Kunitz domain or a Kunitz-type protease inhibitor and a carbohydrate binding module 32-2.

The term *specific binding* in the context of the present invention refers to a property of ligands that bind to their target with a certain affinity and target specificity. The affinity of such a ligand is indicated by the dissociation constant of the ligand. A specifically reactive ligand has a dissociation constant of ≤ 10⁻⁷mol/L when binding to its target, but a dissociation constant at least three orders of magnitude higher in its interaction with a molecule having a globally similar chemical composition as the target, but a different three-dimensional structure.

In the context of the present specification, the term *labelled antibody* or *labelled molecular probe* is used for a molecular probe, particularly an antibody, or nucleic acid probe, being covalently bound to a detectable label. The expression of GATA3, or p53 may be assayed via techniques sensitive to the probe/detectable label chosen, such as PCR, fluorescence microscopy, flow cytometry, mass cytometry, immunohistochemistry or multiplex analyses.

Such detectable labels include those appropriate for flow cytometry detection systems, including, without limitation, small molecule capable of fluorescence in the visible or near infrared spectrum. Examples for fluorescent labels or labels presenting a visible color include, without being restricted to, fluorescein isothiocyanate (FITC), rhodamine, allophycocyanine (APC), peridinin chlorophyll (PerCP), phycoerithrin (PE), alexa Fluors (Life Technologies, Carlsbad, CA, USA), dylight fluors (Thermo Fisher Scientific, Waltham, MA, USA) ATTO Dyes (ATTO-TEC GmbH, Siegen, Germany), BODIPY Dyes (4,4-difluoro-4-bora-3a,4a-diaza-s-indacene based dyes) and the like.

Such detectable labels include those appropriate for fluorescence immunohistochemistry, or in situ hybridisation detection methods, for example, without limitation, octadecyl rhodamine B, 7-nitro-2-1,3-benzoxadiazol-4-yl, 4-acetamido-4'-isothiocyanatostilbene-2,2' disulfonic acid, acridine and derivatives, 5-(2'-aminoethyl)aminonaphthalene-1-sulfonic acid (EDANS), 4-amino-N-(3-[vinylsulfonyl]phenyl)-naphthalimide-3,6-disulfonate dilithium salt, N-(4-anilino-1-naphthyl)maleimide, anthranilamide, BODIPY, Brilliant Yellow, coumarin and derivatives, cyanine dyes, cyanosine, 4',6-diaminidino-2-phenylindole (DAPI), bromopyrogallol red, 7-diethylamino-3-(4'-isothiocyanatophenyl)-4-methylcoumarin, diethylenetriamine pentaacetate, 4,4'-diisothiocyanatodihydro-stilbene-2,2'-disulfonic acid, 4,4'-diisothiocyanatostilbene-2,2'-disulfonic acid, dansylchloride, 4-dimethylaminophenylazophenyl-4'-isothiocyanate (DABITC), eosin and derivatives, erythrosin and derivatives, ethidium, fluorescein, 5-carboxyfluorescein (FAM), 5-(4,6-dichlorotriazin-2-yl)aminofluorescein (DTAF), 2',7'-dimethoxy-4'5'-dichloro-6-carboxyfluorescein, fluorescein isothiocyanate, X-rhodamine-5-(and 6)-isothiocyanate (QFITC or XRITC), fluorescamine, IR-144 (2-[2-[3-[[1,3-dihydro-1,1-dimethyl-3-(3-sulfopropyl)-2H-benz[e]indol2-ylidene]ethylidene]-2-[4-(ethoxycarbonyl)- 1-piperazinyl]-1-cyclopenten-1-yl]ethenyl]-1,1-dimethyl-3-(3-sulforpropyl)-1H-benz[e]indolium hydroxide, inner salt, compound with n,n-diethylethanamine(1:1), CAS No.: 54849-69-3), 5-chloro-2-[2-[3-[(5-chloro-3-ethyl-2(3H)-benzothiazol-ylidene)ethylidene]-2-(diphenylamino)-1-cyclopenten-1-yl]ethenyl]-3-ethyl benzothiazolium perchlorate (IR140), malachite green isothiocyanate, 4-methylumbelliferone, ortho cresolphthalein, nitrotyrosine, pararosaniline, phenol red, B-phycoerythrin, o-phthaldialdehyde, pyrene, pyrene butyrate, succinimidyl 1-pyrene, butyrate quantum dots, Reactive Red 4 (Cibacron Brilliant Red 3B-A), rhodamine and derivatives, 6-carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'tetramethyl-6-carboxyrhodamine (TAMRA) tetramethyl rhodamine, tetramethyl rhodamine isothiocyanate (TRITC), riboflavin, rosolic acid, terbium chelate derivatives, Cyanine-3 (Cy3), Cyanine-5 (Cy5), Cyanine-5.5 (Cy5.5), Cyanine-7 (Cy7), IRD 700, IRD 800, Alexa 647, La Jolta Blue, phthalo cyanine, and naphthalo cyanine.

Such detectable labels relevant for nucleic acid molecular probes include those appropriate for quantitative PCR assay detection methods such as a TAQman, or SYBR green assay, for example, without limitation, tetrachlorofluorescein, and 6-carboxyfluorescien, along with quenchers such as tetramethylrhodamine.

The term *immunohistochemistry* or *IHC* as used herein refers to another desirable method for use to determine a biomarker expression level. It refers to common diagnostic tool utilising specific molecular probes, most often antibodies, to accurately identify expression of structures with reference to tissue morphology, usually a protein. The method often comprises multiple steps, applying primary antibodies (antigen , or biomarker specific), which may be labelled with a detectable label, or followed by secondary ligands, or antibodies bearing detectable labels, most often enzymes which may act on a substrate to form a chromagen which may be visualised by microscopy. To identify tissue structures and cells, a counter stain of a different colour (e.g. haematoxylin) is often applied following chromagen development.

Such detectable labels include those appropriate for molecular probes for immunohistochemistry detection methods, for example, without limitation, streptavidin or avidin (amplified with biotin), or a chromogenic reporter enzyme such as alkaline phosphatase (substrates: Fast Red, nitro blue tetrazolium), or horseradish peroxidase (substrates: 3,3' diaminobenzidine, aminoethyl carbazole).

Another particularly useful means of analysis of biomarker expression encompassed herein is *imaging mass cytometry* (IMC). An exemplary workflow of IMC (Giesen et al., Nature Methods, 2014 Apr;11(4):417-22) comprises preparation of tissue sections for isotope, or metal-chelated antibody labelling using IHC protocols.

Such detectable labels include those appropriate for molecular probes for mass cytometry detection methods, for example, without limitation, the isotope labels listed in Table. 1. Then, tissue samples are positioned in a laser ablation chamber. The tissue is ablated and transported by a gas stream into a time of flight mass spectrometer, such as a CyTOF (Fluidigm) for mass cytometry analysis. The measured isotope signals are plotted using the coordinates of each single laser shot, and a multidimensional tissue image is generated. Single-cell features and marker expression are determined, allowing the investigation of cell subpopulation properties within the analysed tissue.

The term *lymph node metastasis,* as used herein refers to a cancer originating in breast tissue, which has spread to a lymph node (LN). The clinical measure of nodal status quantifies the number and/or locations of LNs with metastases in clinical Tumour-Node-Metastasis staging of breast cancer patients (Cserni, G. 2018, Virchows Arch. 472:697). Patients without metastases in the sentinel LNs are assigned to nodal status N0, which is the best prognosis group. *Lymph node metastasis* encompasses node-positive patients are categorized into levels N1, N2, or N3, which describe increasing numbers of axillary LNs with metastases or increasing distance of involved (non-axillary) LNs from the primary tumour (high node status) and coincide with increasingly worse survival.

The terms *patient sample,* or *lymph node metastasis sample* as used herein can refer to tissue obtained from a patient LN determined to comprise cancer cells (and optionally healthy tissue), for example a biopsy, or excised LN. The sample may be in the form of a cell preparation or lysate, a nucleic acid preparation (particularly isolated mRNA or a cDNA derivative), a tissue section, or embedded within a tumour microarray. A sample format the is particularly useful according to certain thresholds provided by the invention is a histology section obtained a LN metastasis sample embedded in a tumour microarray on the same slide as a control healthy tissue sample as a negative control.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

A first aspect of the invention provides a method to determine the prognosis of a breast cancer patient, by performing an analysis of breast cancer biomarker expression in a tissue sample obtained from a site of lymph node metastasis. The method is suited to predicted the cancer outcomes, or stratifying patients according to what treatments they might be likely to respond to, in order to achieve better outcomes for breast cancer that has spread, or metastasised to a lymph node.

The method comprises the following steps:
Firstly, contacting a lymph node metastasis sample taken from the patient with two distinguishable molecular probes, one which binds specifically to GATA3, and one which binds specifically to p53. Optionally, the probes are applied to a single patient sample, for example, a histology slide, tissue lysate, or nucleic acid preparation. In the case of such co-administration of molecular probes to a sample, biomarker expression is then assessed by measuring the amount of distinct detectable labels associated with each molecular probe. In other embodiments, two molecule probes, one which binds specifically to GATA3, and one which binds specifically to p53, are applied to two different patient samples obtained from the patient, for example, consecutive histology slides, or nucleic acid sample replicates.

This step optionally comprises de-paraffinization, or antigen retrieval steps, removing unbound molecular probe after an appropriate interval of exposure to the sample, or amplification of the signal, for example using an enzyme such as a polymerase, or a chromogenic reporter and substrate, or a secondary antibody.

Secondly, the biomarker expression level is determined using a methodology sensitive to amount, or level of the detectable marker present in the sample, or in a portion of the sample.

The last step of the method is assigning to the patient a prognosis.

The patient is assigned a good prognosis if the patient sample is characterised by a GATA3 expression level greater than or equal to (≥) a GATA3 threshold, and/or a p53 expression level below (<) a p53 threshold. Alternatively, the patient is assigned a poor prognosis if the patient sample is characterised by a p53 expression level ≥ the p53 threshold, and/or a GATA3 expression level < the GATA3 threshold.

The biomarker expression level is determined:
at the level of the whole sample, or a portion of the sample comprising a plurality of cells, and wherein
   - the GATA3 threshold is 100 times ≥ a negative control expression level; and
   - the p53 is threshold is ≥ 8 times a negative control expression level;
and wherein the negative control expression level is the biomarker expression level determined for a negative control sample;
   or
at the level of a single cell for each of a plurality of individual cells present in the sample, wherein
   - the GATA3 threshold is ≥ 20% GATA3 high cells; and
   - the p53 threshold is ≥ 6% p53 high cells,
and wherein a cell is classified as high for biomarker expression if the biomarker expression level of the cell is ≥ 10 times the biomarker expression level of a cell in a negative control sample.

Good or bad prognosis according to this aspect of the invention may refer to a patient outcome, such as survival relative to the average outcome of a cohort of breast cancer patients, or may be relative to patients not assigned the good, or bad prognosis, respectively. Values attached to patient prognosis, for example, overall survival, may be considered to be provisional in that ongoing analysis in larger cohorts, or cohorts with different characteristics or co-morbidities may refine these values based on continuing analysis.

In a particular embodiment of the method according to the method of the first aspect of the invention, good prognosis indicates at least about 60% probability of survival at 300 months, and/or poor prognosis indicates less than about 50% probability of survival at 100 months.

In a particular embodiment of the method according to the method of the first aspect of the invention, good prognosis indicates about 60% probability of survival at 300 months, and/or poor prognosis indicates about 10% probability of survival at 100 months.

The choice of molecular probe according to this aspect of the invention is not particularly limited, as long as it is specific for, or sensitive to expression of the biomarker genes at either the protein or mRNA level. Similarly, many types of detectable label known in the art are suitable for this purpose, paired with a methodology for determining the biomarker expression level that is sensitive to the amount of detectable marker conjugated to the molecular probe specific for p53 and/or GATA3 mRNA, or protein in the sample. Molecular probes of use for this purpose include, but are not limited to, PCR and real time PCR primers, or mRNA hybridising nucleic acid probes, antibodies, or antibody-like molecules, conjugated to fluorescent markers, isotopes, or chromogenic immunohistochemistry reporters.

In particular embodiments, the method provides the use of an antibody, antibody-like molecule and/or nucleic acid probe as molecular probe, particularly wherein the molecular probe is an antibody. Desirable detectable labels according to this embodiment of the invention include, without limit an immunohistochemistry reporter enzyme, an isotope, and/or a fluorescent dye, particularly an antibody bearing a chromogenic enzyme for detection by a standard immunocytochemistry protocol.

Certain embodiments of this aspect of the method according to the invention provide an example of a threshold for use to assign samples analysed in bulk (as opposed to per cell). In other words, the statistical significance of the biomarker expression level is assessed at the level of the whole sample, or a portion of the sample comprising a plurality of cells, and compared to a threshold value, for example derived from a negative control sample analysed previously or concurrently to the sample.

In particular embodiments relevant to measurement of biomarker expression level in a bulk, multicellular assay, for example using antibodies for p53 or GATA3 conjugated to a chromogenic enzyme or isotope, measured by immunohistochemistry, or imaging mass cytometry at the level of biomarker signal intensity across the whole image, the method according to the invention provides the following thresholds:
- Good prognosis is assigned to a patient when a sample has a GATA3 expression level 100 times ≥ a negative control expression level, particularly an expression level 400 times ≥ a negative control expression level, and/or
- Poor prognosis is assigned to a patient when a sample has a p53 expression level ≥ 8 times a negative control expression level.

In one embodiment, negative control expression level is the biomarker expression level determined as specified or a negative control sample, particularly a healthy tissue control sample, for example healthy liver. In some embodiments, the threshold for good prognosis is a GATA3 expression level 100, 150, 200, 250, 300, 350, or 400 times higher expression of GATA3 expression level of a negative control sample.

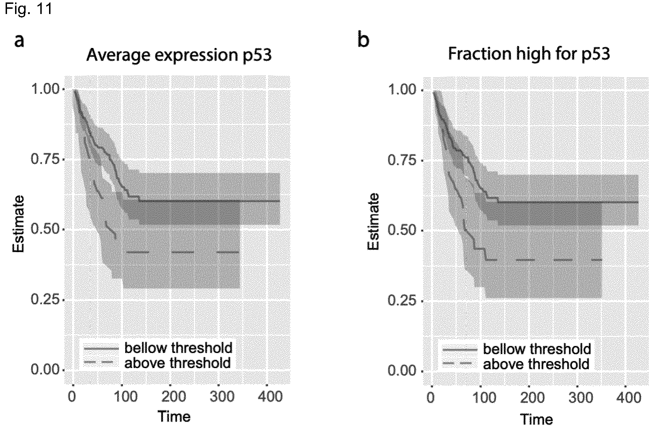

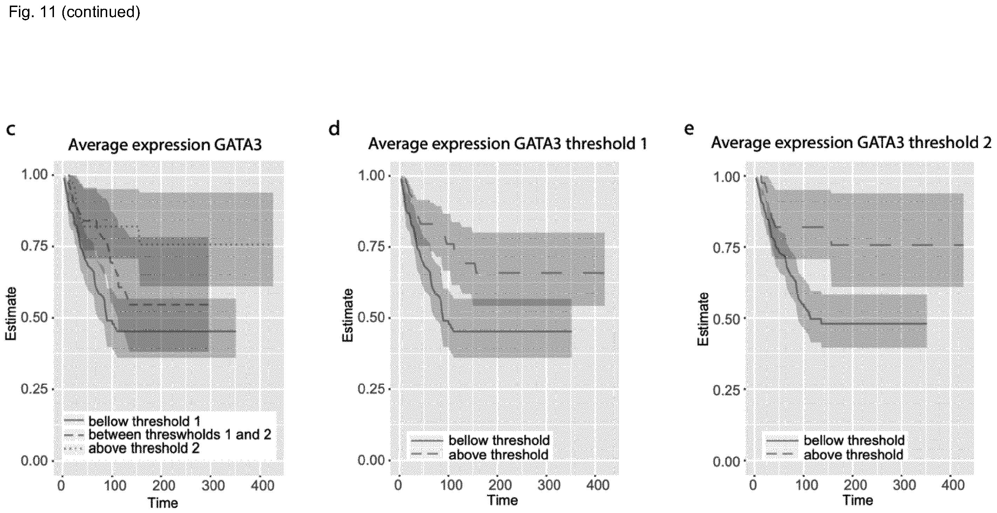

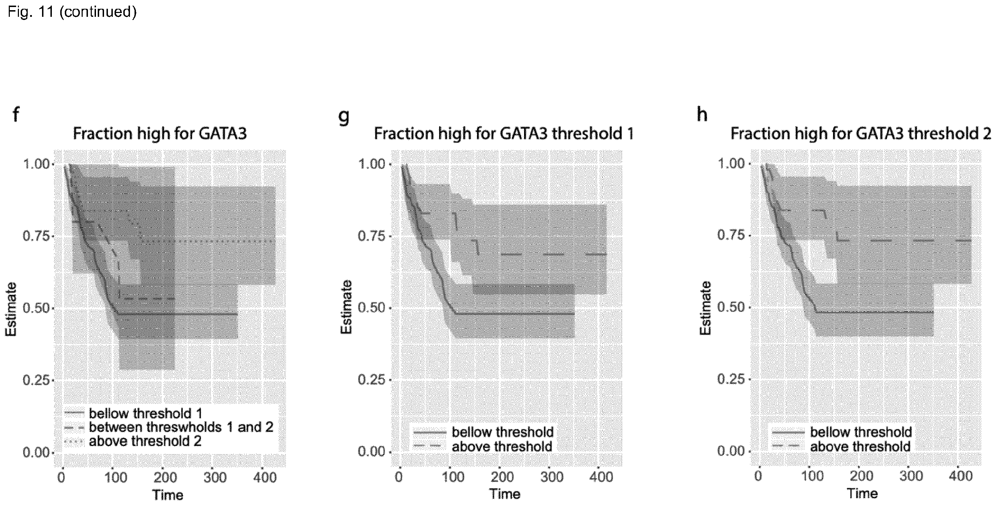

The data presented in Figure. 11 of the examples demonstrates how the specified thresholds are associated with overall survival in a cohort of breast cancer patients analysed by immunohistochemistry according to the method provided by the invention. Separating the patients with a LN GATA3 expression level >100 fold greater than the control from the rest provides a group with statistically significant better survival than the rest. Separating those with >400 fold change, identifies those patients with an even better prognosis. Due to the smaller range of p53 expression observed, only a single example of a threshold is provided for a binary division of patients on the basis of p53 expression and survival.

Another embodiment relates to thresholds distinguishing samples associated with good or poor prognosis based on a determining biomarker expression at the level of single cells. A single cell can be identified by suitable means known in the art, such as forward versus side scatter by flow cytometry, or nuclear staining by a DNA-binding dye such as haematoxylin, Hoechst or DAPI for microscopy. It is understood that the biomarker expression of a plurality of cells must be determined in order to calculate an accurate value representing the proportion of biomarker high cells present in the sample, particularly several hundred or thousands of individual cells. These measurements may be obtained, for example, using antibodies for p53 or GATA3 conjugated to a chromogenic enzyme or isotope, measured by immunohistochemistry, flow cytometry or mass cytometry at the level of biomarker signal intensity per cell as demonstrated in the examples. In similar current clinical practice to assess the biomarker Ki67, a counter stain is used to identify at least 500 individual cells containing nuclei, and positive nuclei are as a proportion of total cells (Dowsett M. 2011 J. Natl. Cancer Inst. 103(22):1656).

According to this embodiment, a cell may be classified high, or positive for p53 or GATA3 expression if the biomarker expression level of the cell is ≥ 10 times a negative control sample. Those skilled in the art will recognise that such a threshold value for biomarker expression may be adjusted depending on what fold change is required to differentiate from noise/the level of background signal present in a cell from a negative control sample (as specified above) contacted with the same molecular probe and detectable label used to examine the patient sample.

According to this embodiment, good prognosis may be assigned to a patient whose sample is determined to have a GATA3 expression level, in other words a proportion of GATA high cells, at or above a threshold of 20% GATA3 positive cells, and particularly ≥ 30% GATA3 positive cells, and/or where fewer than 6% of cells are p53 high. Conversely, bad prognosis may be assigned to a patient when a sample has fewer than 20% GATA3 positive cells, and/or at least 6% p53 high cells.

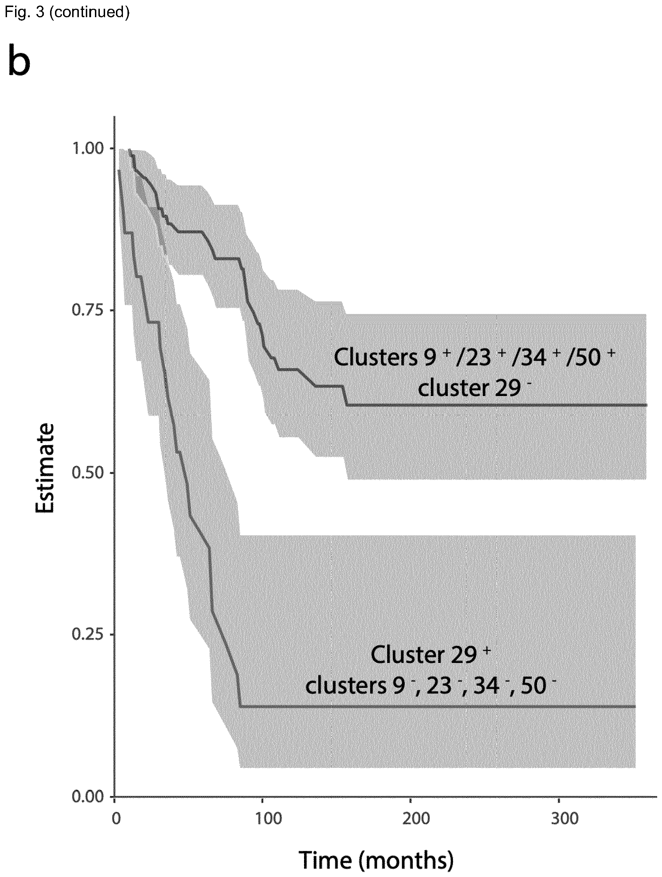

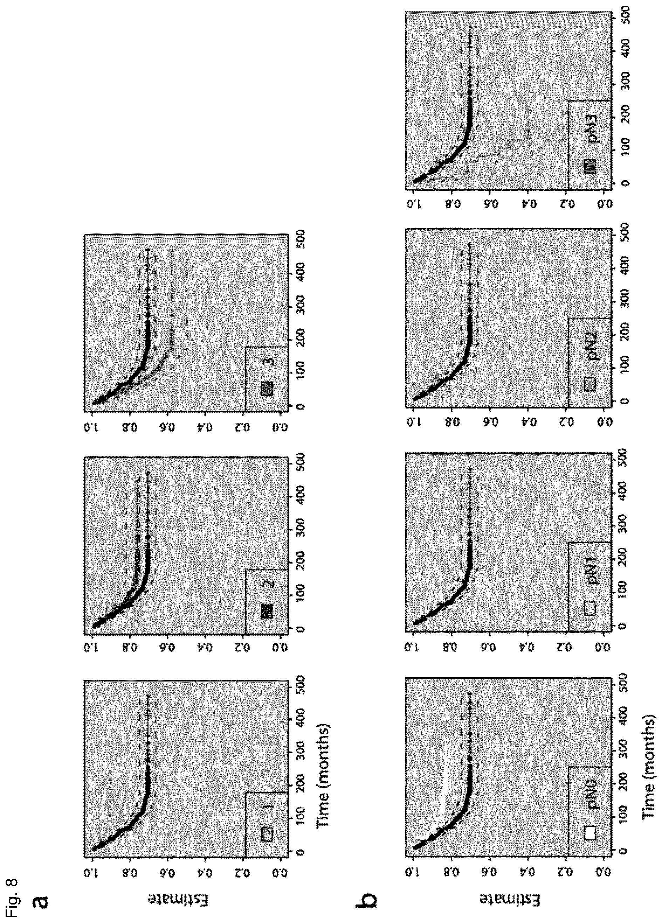

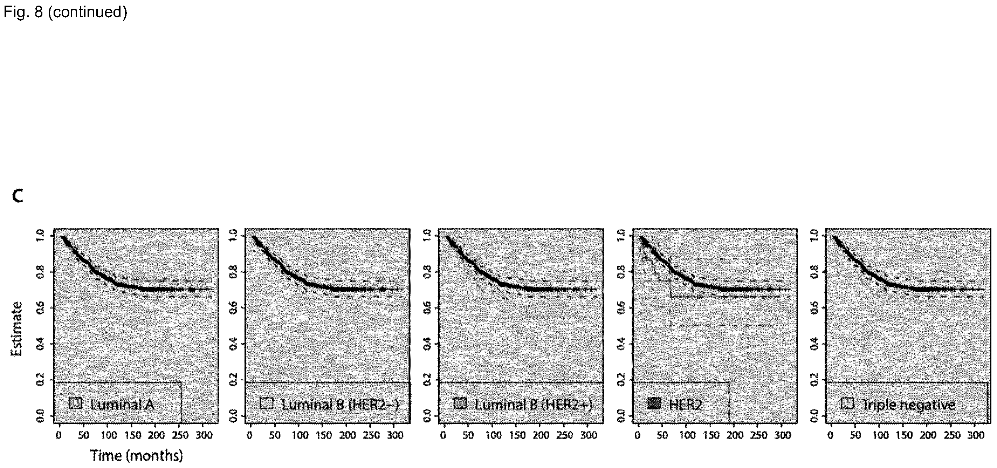

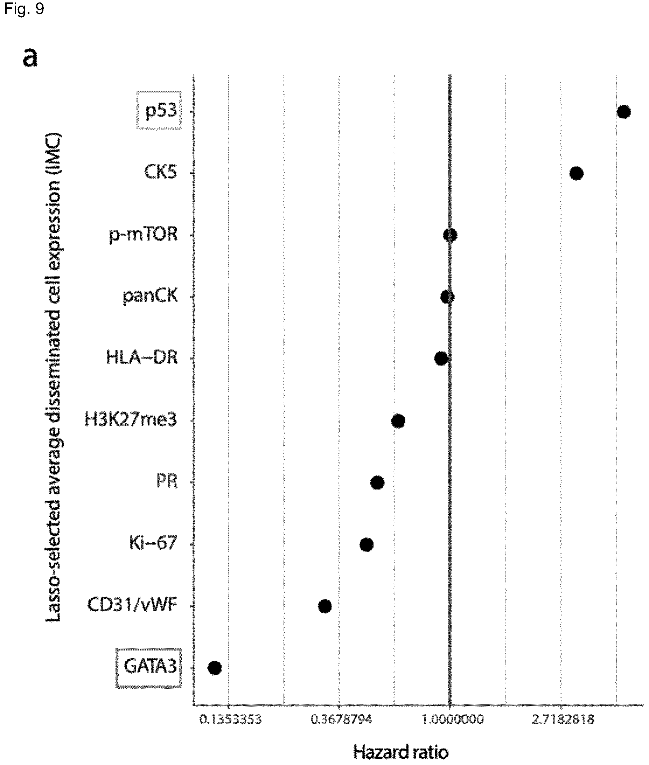

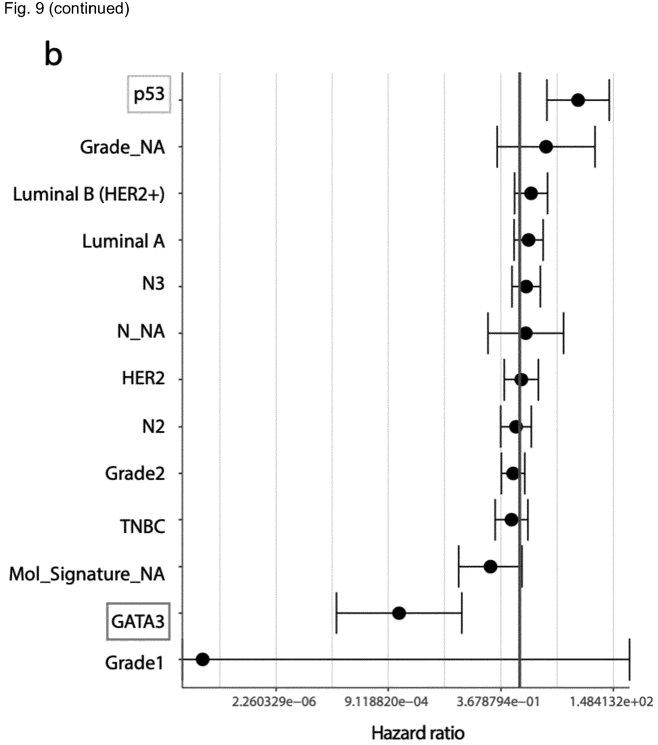

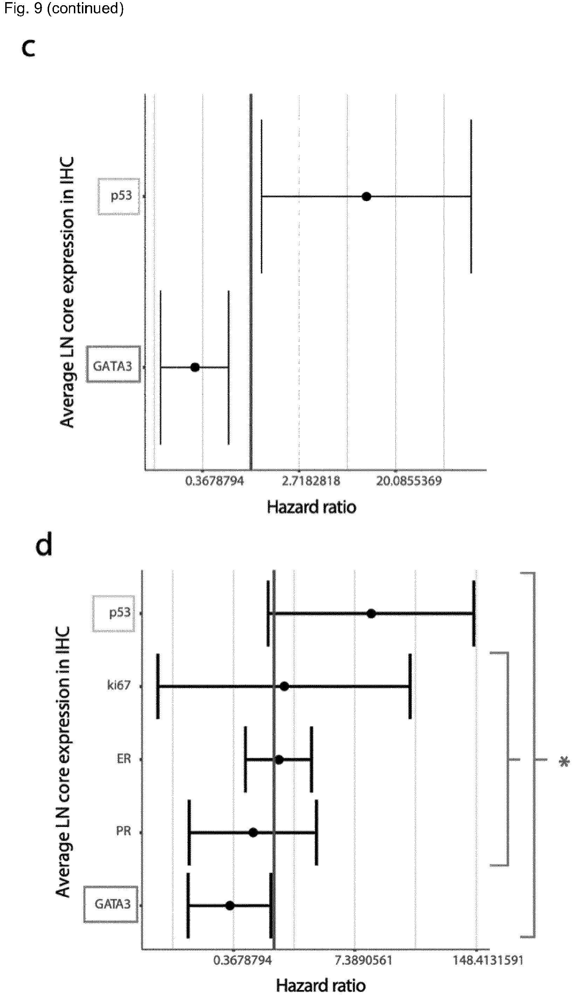

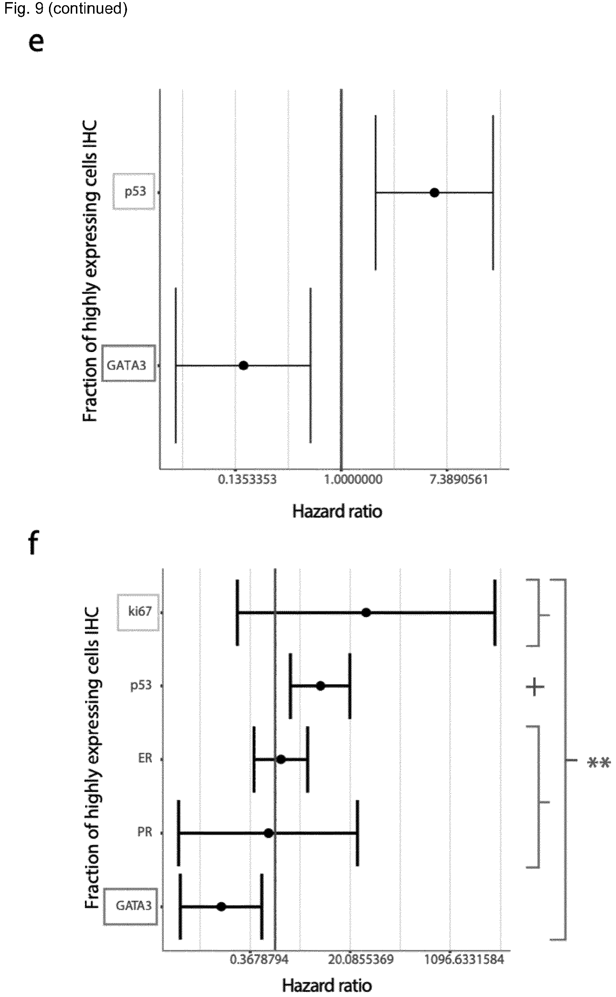

The examples provided herein demonstrate that either p53 or GATA3 expression level alone (Fig. 9) can identify breast cancer patients with differing likelihood of survival using the thresholds specified above, and more specific predictions may be achieved when the two markers are combined, particularly regarding patients with poor prognosis (Fig. 3b) in comparison to current clinical classification of these patients according to primary tumour phenotype (Fig. 8). The wide range of GATA3 expression observed among the cohorts of patients facilitates multiple thresholds according to the invention with increasing likelihood of survival as GATA3 expression increases.

Certain embodiments of the method to predict breast cancer patient outcome relate to use of a means of obtaining information about the expression level of the biomarkers GATA3 and/or p53 that comprises obtaining an image of the ex vivo lymph node metastasis sample, for example, without limits, light microscopy, immunohistochemistry, or mass cytometry used to measure bulk or per cell expression levels.

Other embodiments of the method to predict breast cancer patient outcome according to the invention relate to determining the biomarker expression level in a sample on a histology slide, for example a monolayer of adherent ex vivo lymph node metastasis cells or ex vivo lymph node metastasis cells otherwise immobilised on a solid surface.

Particular embodiments of the method to predict breast cancer patient outcome according to the invention relate to the measurement of biomarker expression level in a sample which retains the original tissue morphology of the sample, encompassing sections of an ex vivo lymph node metastasis sample, for example, a histology section of an excised LN, or a tumour microarray comprising multiple patient samples or controls. A particularly helpful format includes slides bearing an additional healthy liver tissue section embedded under the same conditions alongside the patient sample. Appropriate samples may be obtained by sample preservation methods known in the art, such as cryopreservation, optionally using standard optimal cutting temperature compound, colloidal silica, or paraffin embedding processes.

Further embodiments of the method of particular use in reference to the original tissue morphology sample formats listed above relate to use of a method permitting measurement of the expression level of the biomarkers GATA3 and/or p53 at a cellular, or subcellular level. Measurement methods of particular use for this purpose include imaging mass cytometry at a subcellular resolution, particularly at a resolution of ≤5µm, or even ≤1µm, or immunohistochemistry paired with microscopy analysis.

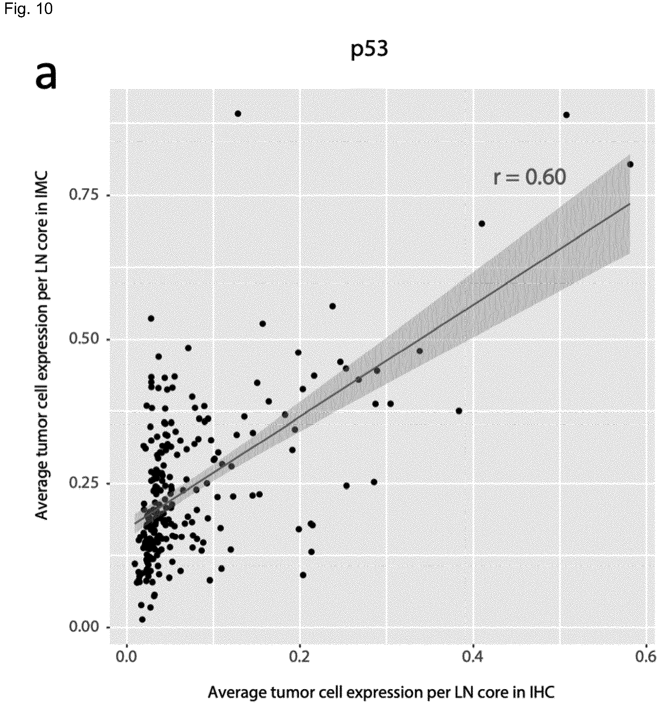

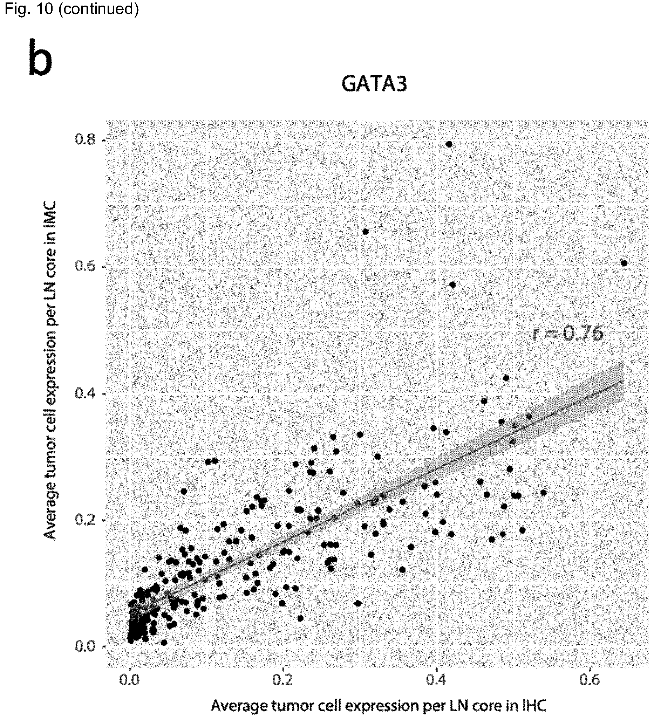

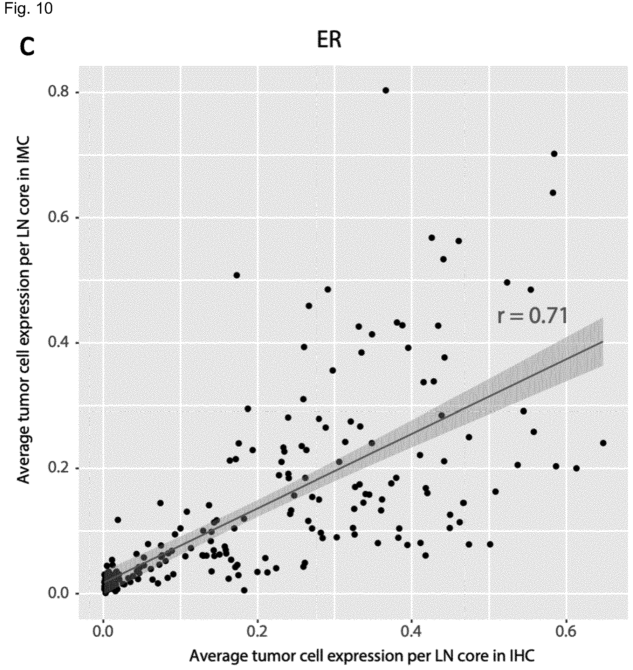

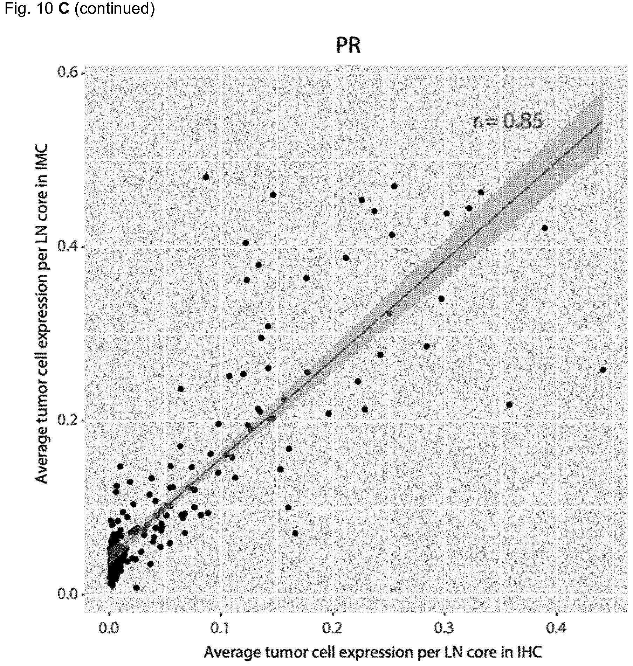

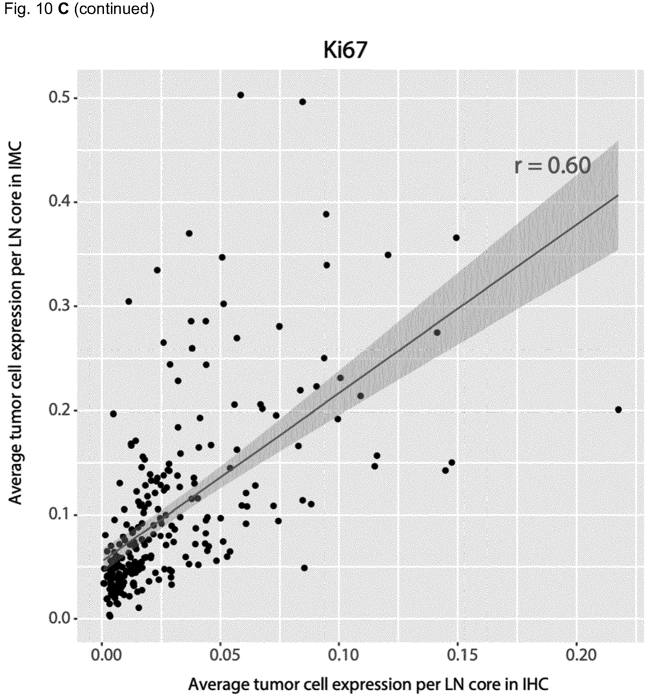

In particular embodiments, the measurement of biomarker expression levels is made using immunohistochemistry methodology paired with microscopy. The data presented in Fig. 10 of the examples demonstrates that equivalent quantitative measurements of GATA3, p53 and standard biomarkers can be achieved using either immunohistochemistry, or mass cytometry measurements of labelled molecular probes contacted with a metastatic LN tissue section sample.

A second aspect of the invention is the use of a kit comprising a molecular probe specific for biomarkers p53, and a molecular probe specific for GATA3, and each molecular probe bearing a detectable label for carrying out the method according to any one of the claims 1 to 8.

A third aspect of the invention is a system for carrying out the method according to any one of the claims 1 to 8, wherein the system comprises a molecular probe bearing a detectable label with specificity for p53, and a molecular probe bearing a detectable label with specificity for GATA3, and a device for analysing the amount of detectable label in a lymph node metastasis sample

The invention is further illustrated by the following examples and figures, from which further embodiments and advantages can be drawn. These examples are meant to illustrate the invention but not to limit its scope.

### Description of the Figures:

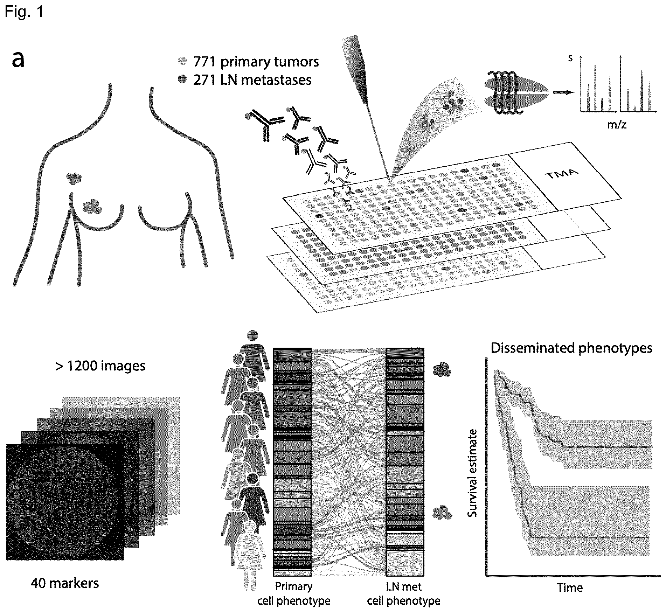

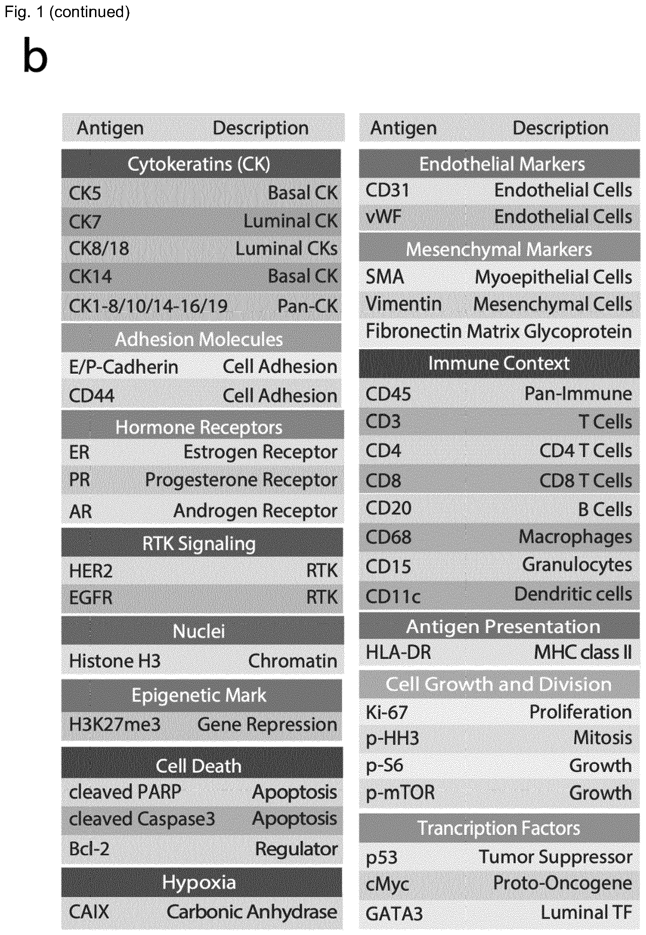

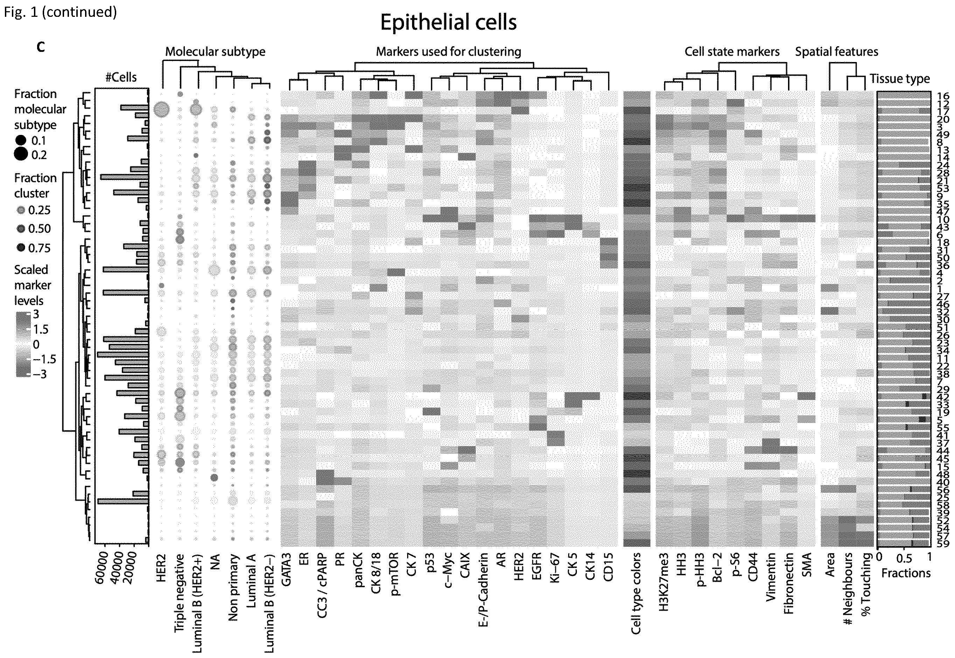

- Fig. 1: shows **a** workflow of single-cell phenotypes of primary breast cancers and LN metastases characterized by imaging mass cytometry. **b** Antigens recognized by the breast cancer-focused antibody panel. **c** Heatmaps of z-scored mean marker expressions of the epithelial phenotype clusters identified using PhenoGraph. Markers used for clustering, additional markers of interest, and spatial single-cell features are indicated. (Left) fractions of cells in each cluster found in primary breast cancers and lymph node metastases. Relative fractions of cells shared between the clusters and clinically assigned molecular subtypes as bubble plots (right), and absolute cell counts displayed in bar plots.

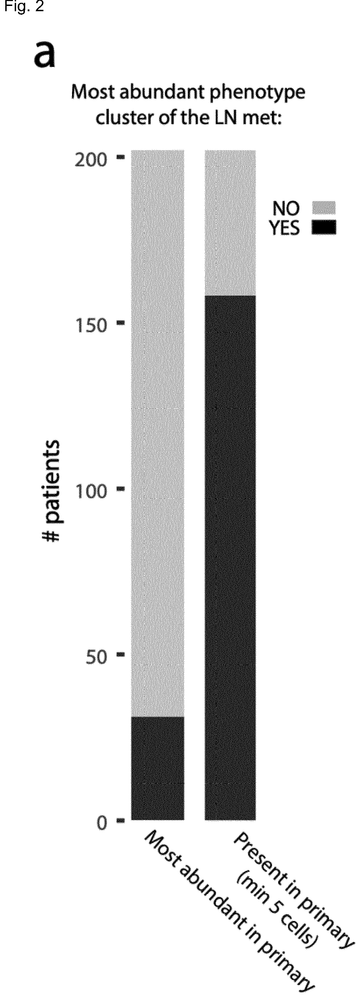

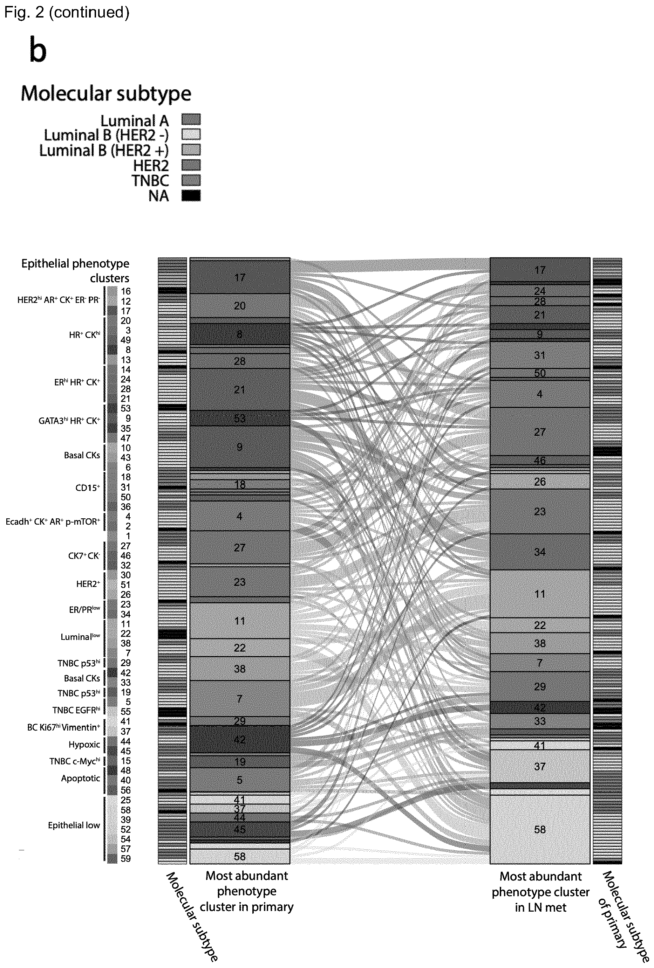

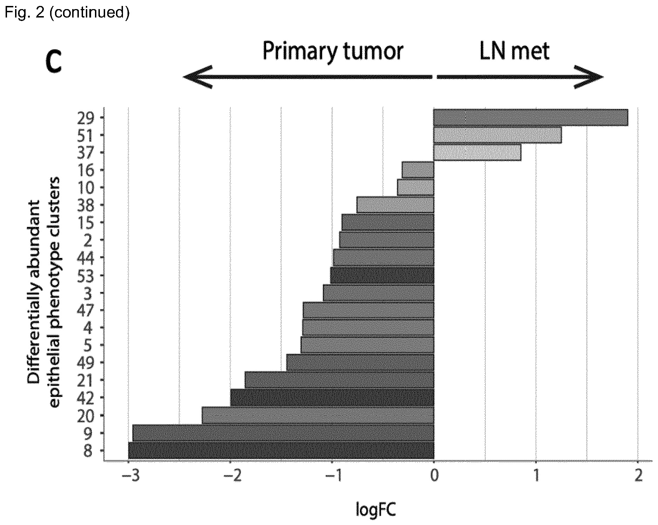

- Fig. 2: shows single-cell phenotypes differ in paired primary tumours and lymph node metastases. **a** Bars indicate the number of patients for which the most abundant disseminated single-cell phenotype matches the predominant phenotype in the primary tumour (left) or is identified in the primary tumour in at least 5 cells. **b** Flow diagram relating the predominant primary tumour phenotype (left) to the most abundant phenotype identified in the matched LN metastasis (right). Blocks are shaded according to the most abundant tumour phenotype, and the block sizes represent the number of patients with the given predominant phenotype in the primary tumour or LN metastasis. Outermost vertical bars indicate the fraction of patients of each block clinically assigned to the distinct molecular subtypes based on histologic stratification of the primary tumour. The connecting lines between the primary tumours and LN metastases are coloured according to the predominant phenotype of the primary tumour and their width represents the number (order not meaningful) of patients shared between blocks. **c** Log fold changes in frequency of tumour phenotypes between the matched primary breast and LN site (p < 0.01) by paired-design differential abundance testing using edgeR.

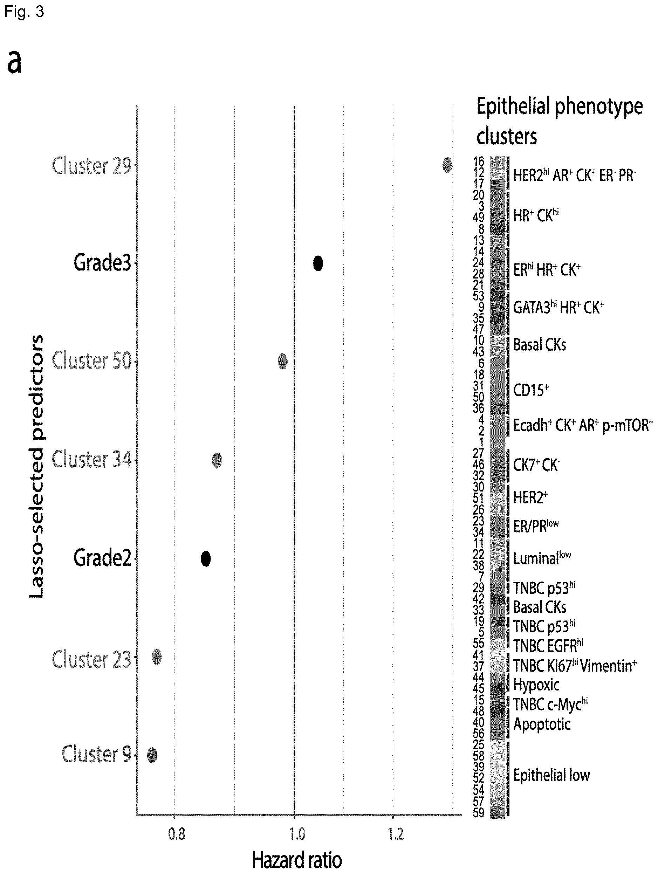

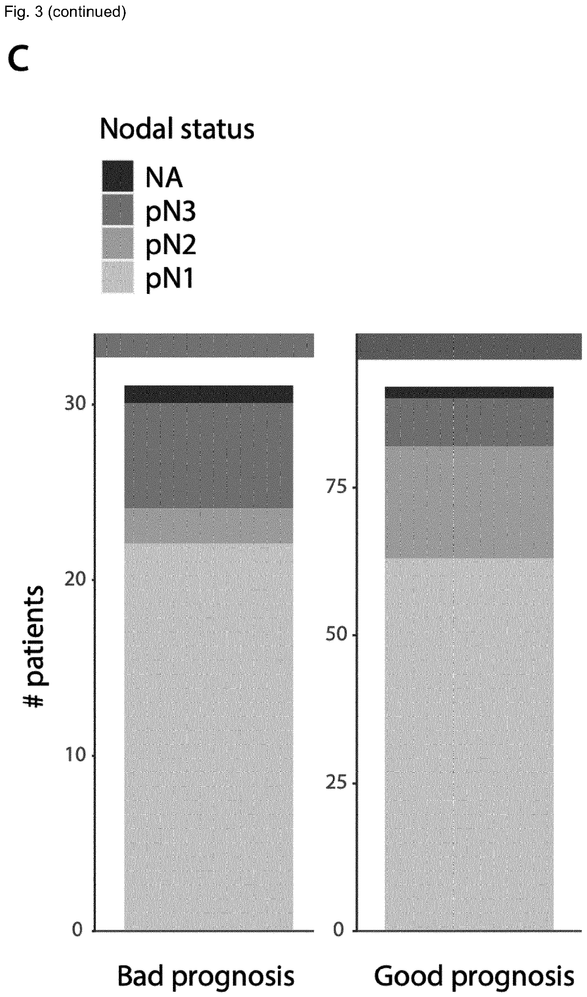

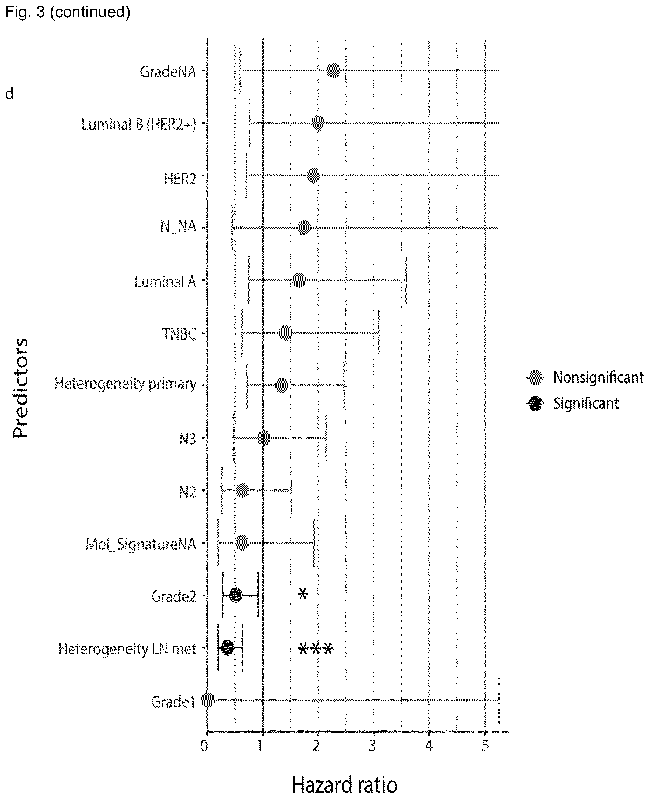

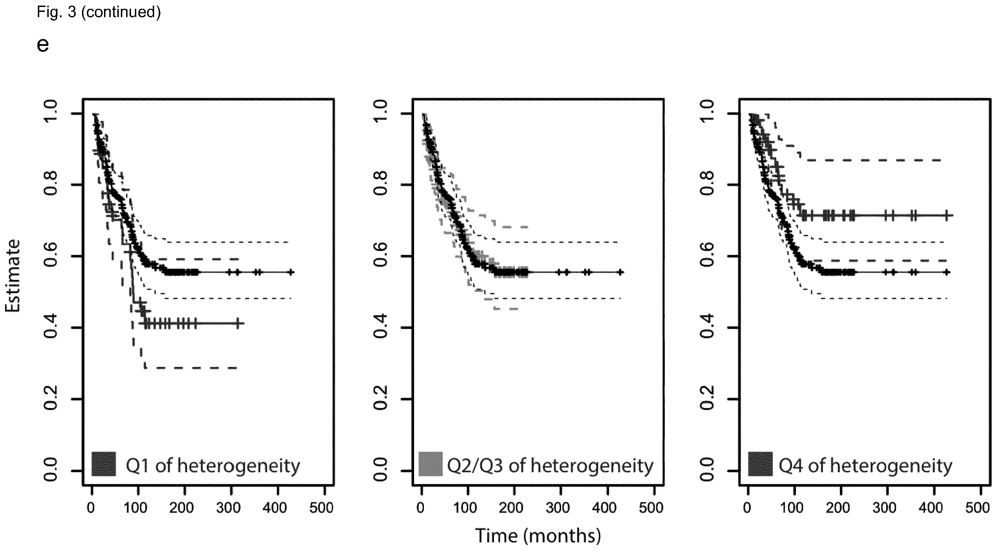

- Fig. 3: shows disseminated tumour single-cell phenotypes and heterogeneity are associated with patient survival. **a** Hazard ratios of stratifying predictors selected by a lasso-regularized Cox proportional hazards model for overall survival. Inputs were categorical presence or absence of all tumour cell phenotypes in the LNs (threshold 5 cells) and the clinical classifications of the patients (primary tumour grade, molecular subtype of primary tumour, and nodal status) for the 213 patients with LN metastasis samples and survival information available. Hazard ratios are displayed without confidence intervals. **b** Kaplan-Meier survival curves for patients with exclusively good or bad prognosis predictive phenotypes as identified in panel a in the LN metastases (good prognosis n= 92, bad prognosis n=31). **c** Bar plot of the number of patients of each nodal status in each group of the survival curves in panel b. **d** Hazard ratios and confidence intervals returned by a Cox proportional hazards model for overall survival of the 166 patients with matched primary tumour and LN metastasis samples and survival and clinical information. Model predictors were primary tumour and intra-lesion LN metastasis heterogeneity, and clinical patient categories (primary tumour grade, molecular subtype of primary tumour, and node status). The reference groups for the categorical predictors are grade 3, nodal status N1, and molecular subtype Luminal B (HER2-). **e** Kaplan-Meier survival curves of the patients with LN metastases of different heterogeneity quartiles. The black curve represents the survival across all patients with LN metastasis samples. Dashed lines indicate the 95% confidence intervals.

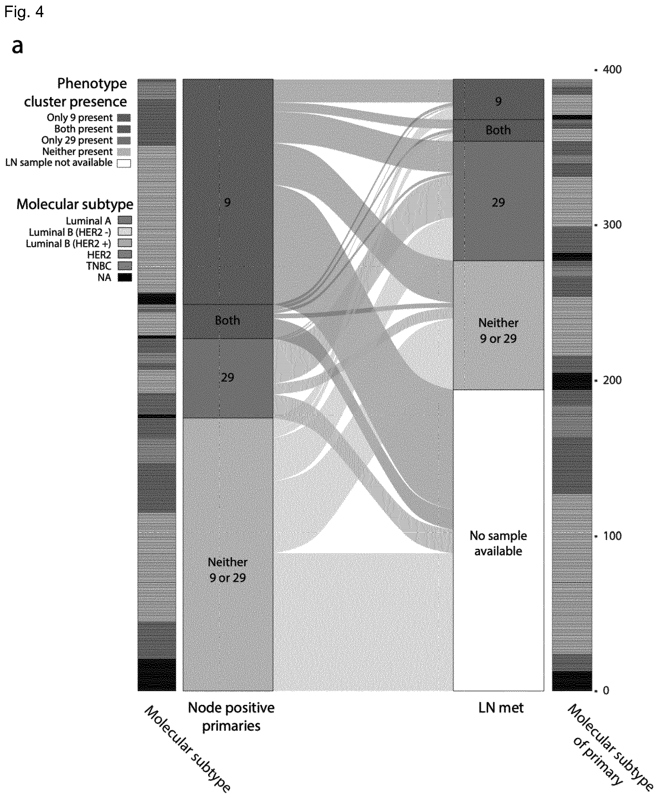

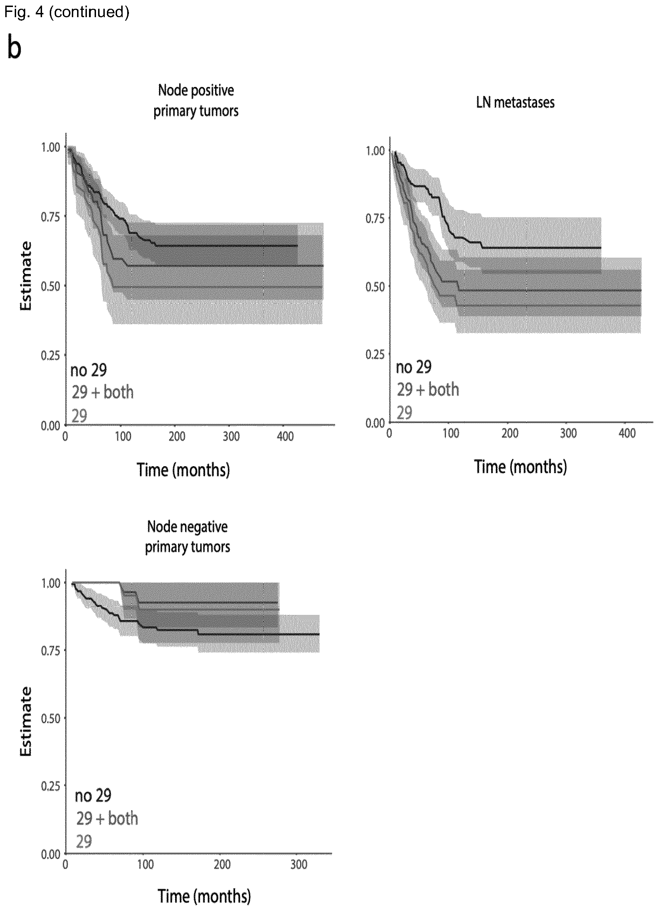

- Fig. 4: shows primary tumour biomarkers of systemic disease. **a** Flow diagram of primary tumours and corresponding LN metastases in which the disseminated single-cell phenotypes with the greatest opposing impact on patient survival (epithelial clusters 9 and 29) were identified (threshold 5 cells). Inner bar blocks represent the number of primary tumours (left) and LN metastases (right) with indicated clusters. Outer vertical bars indicate the fraction of patients of each block clinically assigned to the distinct molecular subtypes based on histologic stratification of the primary tumour (order not meaningful). **b** Kaplan-Meier survival curves of patients with indicated primary tumours or LN that contained cells of cluster 29, that contained cells from both clusters 9 and 29, and that did not contain cluster 29 cells.

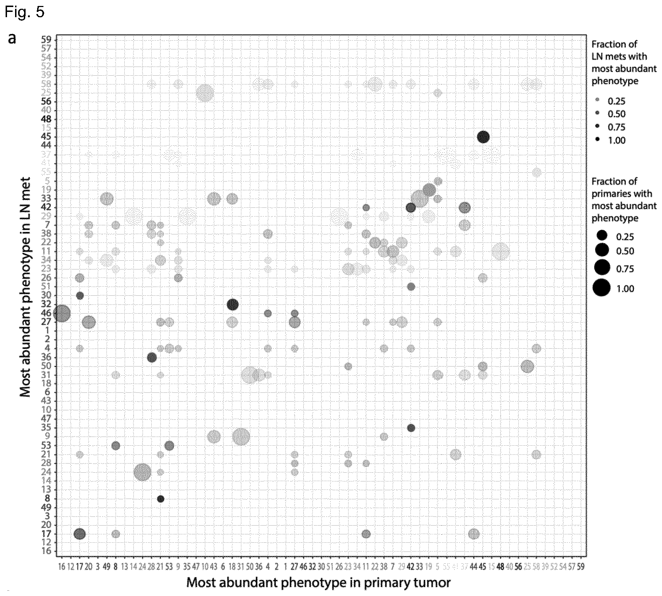

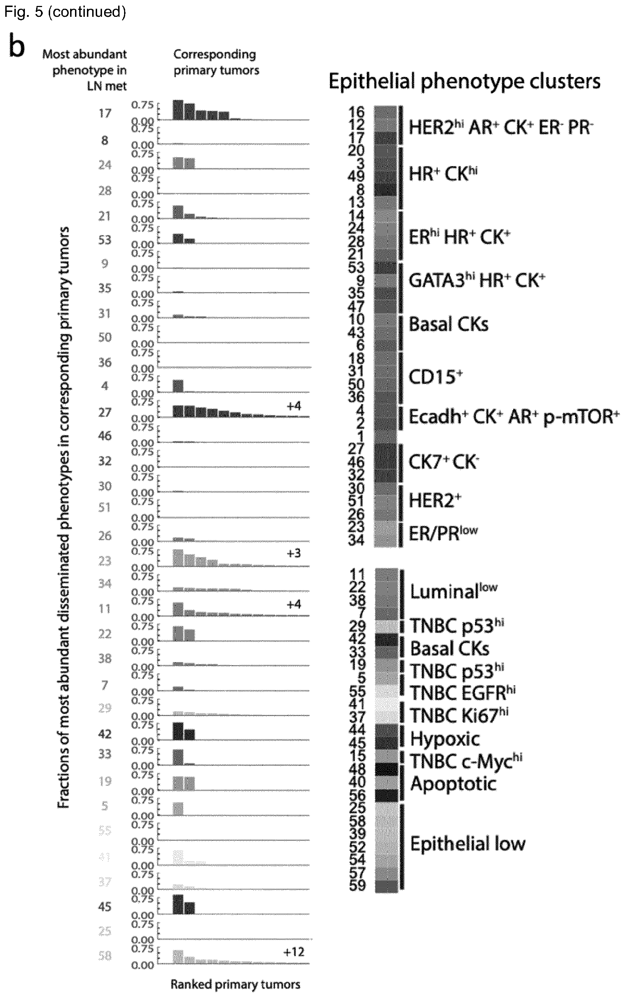

- Fig. 5: shows **a** Bubble plot relating the most abundant single-cell phenotype of the primary tumour to the predominant disseminated phenotype identified in the matched LN metastasis. Circle size is the fraction of primary tumours and opacity is the fraction of LN metastases with a given predominant phenotype. **b** Patients with a given most abundant disseminated tumour phenotype (y-axis), the fractions of tumour cells of the same phenotype in the corresponding primary tumours (x-axis) are displayed in order of decreasing prevalence and coloured by predominant disseminated phenotype. **c** Cumulative density function of patients with a given fraction of the disseminated cell phenotype in the primary tumour.

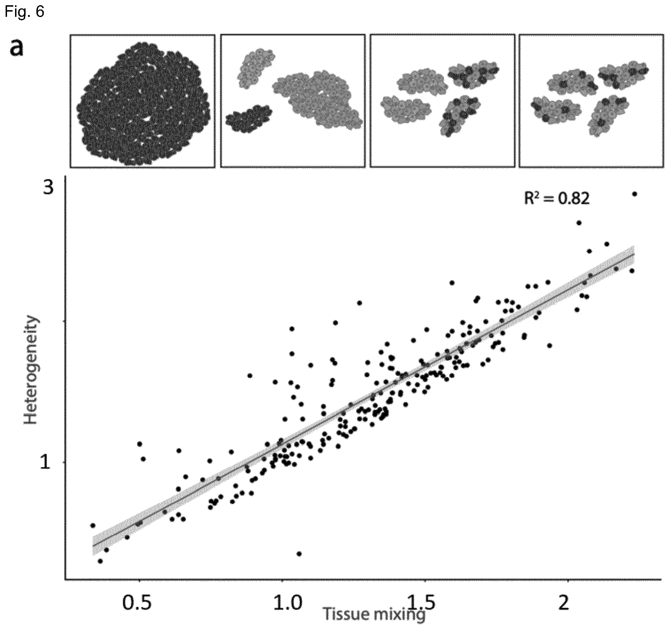

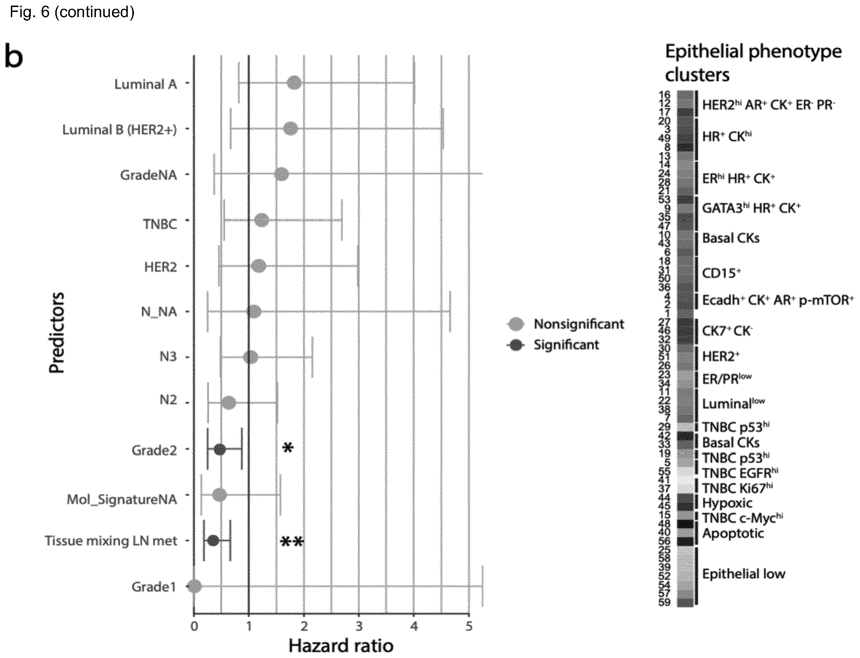

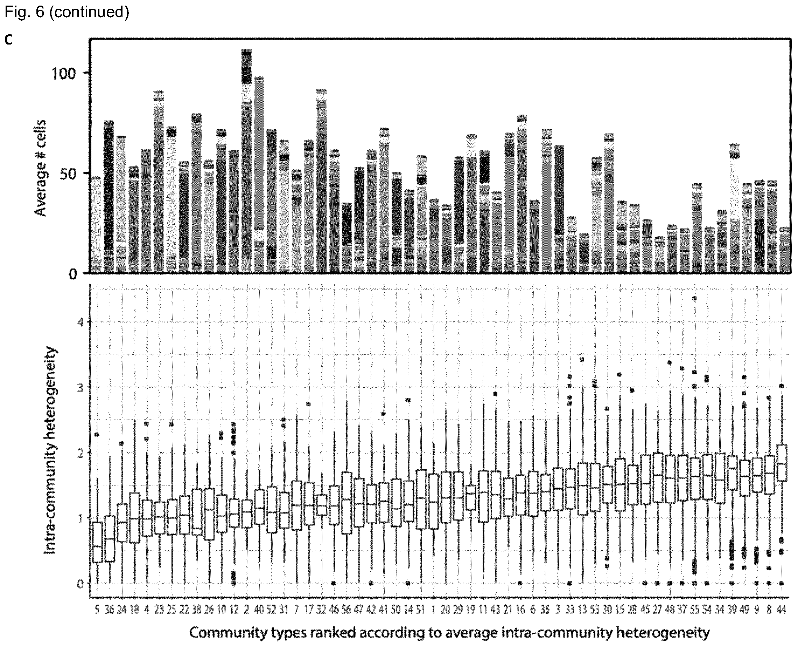

- Fig. 6: shows **a** correlation between intra-lesion heterogeneity (Shannon entropy calculated using all tumour cells) and tissue mixing of different phenotypes (average intra-community Shannon entropy) in the LN samples n=271. **b** Hazard ratios and confidence intervals returned by a Cox proportional hazards model for overall survival of 213 patients with survival information available. Inputs were the mixing scores of the LN metastases, and clinical patient categories (primary tumour grade, molecular subtype of primary tumour, and node status). The reference groups for the categorical predictors are grade 3, nodal status N1, and molecular subtype Luminal B (HER2 negative). **c** Bar plot of average number of cells of the single-cell phenotype clusters in each community type ranked by average intra-community heterogeneity, quantified using Shannon entropy.

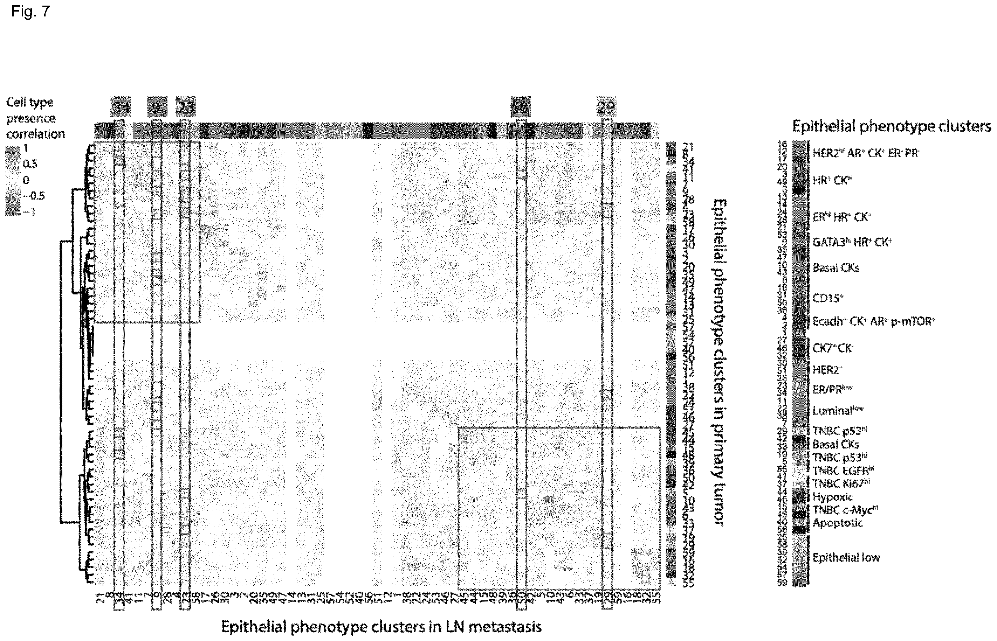

- Fig. 7: shows heatmap of tumour single-cell phenotype cluster correlations between the matched LN metastases (x-axis) and primary tumours (y-axis) based on categorical presence/absence (threshold 5 cells) of epithelial phenotype clusters in each of the tissues. Squares mark the correlation structure between the good-prognosis phenotypes of disseminated cells and luminal, hormone receptor-positive cell type clusters present in the primary tumour, and between bad-prognosis phenotypes of disseminated cells and non-luminal cells present in the primary tumour. Vertical black bars highlight the prognostic phenotypes and the black boxes within them mark the phenotypes in the primary tumour that were selected by a lasso-regularized generalized linear model to best predict the presence of a given disseminated phenotype (boxes within the squares are positive predictors, the rest are negative predictors).
- Fig. 8: shows Kaplan-Meier survival curves n=614 patients for the clinically assigned categories based on **a** pathology grade of the primary tumour, **b** nodal status, and **c** molecular subtype of the primary tumour. Dashed lines indicate the 95% confidence intervals. The black curves plot the survival of all patients.
- Fig. 9: shows **a** Hazard ratios of stratifying predictors selected by lasso-regularized Cox proportional-hazards model for overall survival. The model was provided with the average tumour cell expression levels of all markers in the LN metastases (221 patients). Hazard ratios confidence intervals are not meaningful after feature selection. **b** Hazard ratios and confidence intervals of Cox Proportional-Hazards model for overall survival provided with the average expressions levels of p53 and GATA3 in the LN metastases, as well as all clinical features of the patients (primary tumour grade, molecular subtype of primary tumour and nodal status) (221 patients). The reference groups for the categorical predictors are grade 3, nodal status N1 and molecular subtype Luminal B (HER2-). **c-f** Cox Proportional-Hazards models based on IHC stained markers of interest. Models including average tumour cell marker expressions in the LN metastases for c p53 and GATA3 and d the clinically established markers (ER, PR, Ki-67) and p53 and GATA3. Models based on fractions of highly expressing tumour cells in the LN metastases for **e** p53 and GATA3 and **f** the clinically established markers (ER, PR, Ki-67) and p53 and GATA3. Vertical lines and significance stars indicate likelihood ratio tests for comparison of nested models. The smaller models include only the clinical markers (ER, PR, Ki-67) and the bigger models additionally p53 and GATA3.
- Fig. 10: shows **a** Scatterplot and Pearson correlation of average tumour cell expression levels of p53 between sections of the same LN core quantified based on IHC or IMC signal. **b** Scatterplot and Pearson correlation of average tumour cell expression levels of GATA3 between sections of the same LN core quantified based on IHC or IMC signal. **c** Scatterplots and Pearson correlations of average tumour cell expression levels of the clinical markers between sections of the same LN core quantified based on IHC or IMC signal.
- Fig. 11: shows Kaplan-Meier survival curves for patients bellow or above the defined thresholds for distinct patient prognosis. Thresholds for p53 expression based on **a** average expression levels across all tumour cells of a LN metastasis core (threshold: >8 fold change to a negative control sample) and **b** fraction of highly expressing cells in a LN metastasis core (threshold: >6% of cells with high expression). **c-e** Thresholds for average expression levels across all tumour cells of a LN metastasis core for GATA3 (threshold 1: >100 fold change to a negative control sample; threshold 2: >400 fold change to a negative control sample). **f-g** Threshold for fraction of highly expressing cells for GATA3 (threshold 1: >20% of highly expressing cells per LN metastasis core; threshold 2: >30% of highly expressing cells).
- Table 1.: list of antibody clones and labels used in this study.

### Examples

### Example 1: Single-cell phenotypes differ in paired primary tumours and lymph node metastases

The single-cell phenotypic landscape of primary tumour tissues were compared with disseminated cells in matched LN metastases in a cohort of breast cancer patients matched to clinical and long-term survival information (Figure 1a). The sample set included tissues from 771 primary tumours and from 271 LN metastases collected prior to any treatment. Tissue microarrays were stained with a breast cancer-specific panel of 40 antibodies including antibodies to clinically established markers ER, PR, HER2, and Ki67, signalling proteins, epigenetic factors, oncogene products, and markers of treatment resistance and invasive potential as well as components of the tumour microenvironment such as endothelial, mesenchymal, and immune cell types (Figure 1b). High-dimensional images were acquired using imaging mass cytometry, segmented into >2 million single cells, and analysed based on single-cell marker expression and cellular organization within the tissue architecture.

Single-cell phenotypes were identified in all 771 primary tumour and 271 metastatic LN tissues based on high-dimensional single-cell marker expression, separated into epithelial and non-epithelial cells and then identified single-cell phenotypes using unsupervised PhenoGraph clustering on each group with a selected set of markers. Within the epithelial cell group, 59 clusters of phenotypically similar tumour cell clusters were identified; most were found at both the primary breast and metastatic LN sites (Figure 1c). These epithelial cell phenotypes reflected all clinical molecular subtypes split into more finely grained tumour cell phenotypes. Multiple non-luminal (luminal CK negative) and triple-negative (ER-, PR-, and HER2-) subtypes were distinguished by varying expression of basal CKs, cMYC, EGFR, p53, CAIX, Ki67, and CD15 (Figure 1c). Although commonly used to identify myeloid cells, CD15 has previously been detected on epithelial cells in breast cancer. Luminal cell phenotypes (CK7+, CK8/18+, or panCK+ but negative for the basal CKs 5 and 14) were identified with and without ER, PR and/or HER2 expression and with varying levels of the luminal CKs, E/P-Cadherin, p-mTOR, GATA3, and AR (Figure 1c). Bcl-2, which has been proposed as a prognostic breast cancer marker, strongly correlated with both GATA3 and ER expression in our analysis (Spearman correlations for GATA3 and Bcl-2 of 0.67 and for ER and Bcl-2 of 0.67) but added little discriminatory phenotypic information. A correlation between AR expression and HER2 expression, particularly notable in the clinical HER2 molecular subtype, which is typically negative for ER and PR expression but was characterized in the current cohort by high levels of AR (Figure 1c). None of the phenotypes were found exclusively in LN metastases, but cell phenotypes with elevated expression of the ER, PR, GATA3, Bcl-2, p-mTOR, and luminal CKs were predominantly identified in primary tumour tissues (Figure 1c).

To relate disseminated tumour cells to the single-cell phenotypes identified in the primary tumour, primary tumours were compared with matched LN metastases for 203 patients, revealing substantial differences in the tumour cell phenotypes found at the two sites of the same patient. The most abundant cell phenotype cluster differed between primary tumour and LN metastasis in 84% of cases (Figure 2a, b, Figure 5a). Although the most abundant disseminated phenotype cluster was detected as a subpopulation in 78% of the primary tumours, despite limited sampling of a single primary tumour core (Figure 2a, Figure 5a, b and c), the disseminated phenotype made up less than 20% of the primary tumour sample in 75% of patients (Figure 5c). Furthermore, disseminated cell phenotypes varied substantially even among patients with primary tumours that contained similar phenotypes (Figure 5a).

Clinically established markers varied greatly between primary tumours and matched LN metastases. Certain hormone receptor-positive cell phenotypes (e.g., epithelial cluster 20) formed the bulk of some primary tumours but were not detected in LN metastases; the disseminated cells of these primary tumours were frequently negative for the hormone receptors (e.g., epithelial cluster 27, Figure 2b). Other cell phenotypes, such as those with high HER2 expression (e.g., epithelial cluster 17) of the HER2 or Luminal B (HER2+) clinical subtype, dominated both the primary tumour and corresponding LN metastasis in most cases (Figure 2b). However, some HER2+ primary tumours resulted in predominantly HER2- or even luminal hormone receptor-positive HER2- LN metastases (pairs of predominant primary and predominant LN cell phenotype: 17 and 21, 17 and 4, and 17 and 11, Figure 2b; 'phenotype pairs' hereafter). Conversely, in some cases predominantly luminal HER2- primary tumours yielded HER2+ LN metastases (phenotype pairs 8 and 17, and 11 and 17, Figure 2b). Although some hormone receptor-positive primary tumours resulted in hormone receptor-positive LN metastases, in certain cases the dominant disseminated cell phenotype expressed luminal CKs but not hormone receptors (e.g., phenotype pair 20 and 27) or showed characteristics of non-luminal triple-negative breast cancer (TNBC) (e.g., phenotype pairs 21, 53 or 9 and any of 29/42/37/58, Figure 2b). Most non-luminal triple-negative primary tumours, but also various luminal primary tumours, resulted in predominantly non-luminal triple-negative LN metastases and, in rare cases, non-luminal TNBC primary tumours gave rise to LN metastases with luminal expression patterns (e.g., phenotype pairs 29 and any of 27/34/22, and phenotype pairs 1 or 5 and any of 11/37/7, Figure 2b).

The phenotypic variability between primary tumours and LN metastases was also apparent in correlation analyses between tumour single-cell phenotype clusters in these tissues. Strong positive correlations existed among similar phenotype clusters within primary or LN tissues, as previously observed in primary tumours (Jackson, H. W. 2020 Nature, 578:615). Between matched primary and LN metastatic tissue, however, positive correlations occurred between different phenotypes, including between differing phenotypes characterized by clinically established molecular markers. In accordance with this phenotypic heterogeneity, grouping patients based on either the cluster composition of the primary tumours or that of the LN metastases resulted in substantially different groups. Although the primary tumour groups showed expected enrichments for the clinically assigned molecular subtypes and grades, neither the primary tumour-defined groups nor the LN metastasis-defined groups were associated with nodal status.

Differential abundances of tumour single-cell phenotype clusters were assessed in the primary tumours and matched LN metastases with the goal of identifying tumour cell phenotypes with a propensity to disseminate. Cells with high levels of ER, PR, GATA3, and luminal CKs were more abundant in primary tumours (epithelial clusters 3, 8, 9, 20, 21, 47, 49, 53; Figure 2c) as were tumour cells with low luminal expression patterns (epithelial clusters 2, 38), with high expression of p-mTOR (epithelial cluster 4), as well as some triple-negative cell phenotypes including EGFR+ (epithelial cluster 5), c-MYC+ (epithelial cluster 15), and hypoxic cells (epithelial cluster 44; Figure 2c). In the LN metastases, the more abundant cells included HER2+ cells with otherwise low marker expression (epithelial cluster 51) and two types of triple-negative cell phenotypes, one expressing high levels of p53 (epithelial cluster 29) and the other highly proliferative and expressing Vimentin (epithelial cluster 37; Figure 2c). This is consistent with the fact that triple-negative and HER2+ tumour cells are considered aggressive. The most abundant phenotypes in LNs differ from the most abundant phenotypes in the corresponding primary tumours indicating that dissemination-prone cell phenotypes are typically present at low proportions in the primary tumours, consistent with the observation that the predominant phenotype in LN metastases rarely coincided with that in the primary tumour (Figure 2a, Figure 5). Thus, extensive variability exists among the predominant phenotypes of matched primary tumour and metastatic LN tissues. However, the most abundant disseminated tumour phenotype, often with molecular features that deviated from the clinical classification, was frequently identified as a subpopulation in the matched primary tumour.

### Disseminated tumour phenotypes are associated with patient survival

The dissemination of tumour cells to the LNs, reflected by the nodal status, is an important prognostic factor, but current clinical evaluation does not take into account the phenotypes of the disseminated cells. To determine which disseminated tumour phenotypes impact survival and how these phenotypes relate to clinical status, a lasso-regularized Cox proportional-hazards model was applied inputting categorical presence or absence information of all tumour cell phenotypes in the LNs (threshold for presence = 5 cells) as well as the clinical classifications of the patients (primary tumour grade, molecular subtype of primary tumour, and nodal status). The model identified a predictive set of disseminating cell phenotypes that stratify the overall survival of node-positive patients; primary tumour grade was the only selected stratifying feature among the clinical classifications (Figure 3a). Disseminating cells that were **non-luminal triple-negative** and that expressed high levels of **p53** (epithelial cluster 29) were associated with **poor patient survival** (Figure 3a). Since cells of cluster 29 were more abundant in LN metastases and rarely predominant in primary tumours (Figure 2), these may represent dangerous tumour cells with an increased metastatic potential, but only present as a small subpopulation in the primary tumours. Conversely, hormone receptor-positive disseminated cells were associated with better node-positive patient outcome (in order of increasing effect size: epithelial clusters 34, 23, and 9) (Figure 3a). The largest positive prognosis effect was associated with epithelial cell cluster 9 (Figure 3a). These cells are **hormone receptor-positive,** express high levels of **GATA3,** and were more abundant in primary tumour tissues than LNs (Figure 2c, 3a). This **good-prognosis phenotype** may occasionally be drained to LNs but may have only minor invasive potential or may be **sensitive to standard therapy.** Patients with LN metastases consisting of such better prognosis phenotypes **may not require aggressive chemotherapy.**

To illustrate the prognostic value of these predictive single-cell phenotypes, patients with disseminated cells of exclusively bad- or exclusively good-prognosis predictive phenotypes were analysed. Patients with LN metastases containing cells from cluster 29, but not the good-prognosis clusters 9, 23, 34, and 50, had a drastically worse prognosis than those with the opposite pattern (Figure 3b). While the worse-prognosis patients were slightly enriched for nodal status N3 and the better prognosis patients for N2, about 70% of patients in both groups were of nodal status N1 and could therefore not be distinguished on this basis (Figure 3c). Therefore, multiplexed single-cell phenotyping of the disseminated tumour cells can provide prognostic information not evident in nodal status alone.

### Intra-lymph node metastasis heterogeneity is associated with patient survival

Cellular heterogeneity of the metastatic lesion was assessed for association with patient prognosis. Tumour cell heterogeneity was quantified using Shannon entropy and assessed for association with overall patient survival using a Cox proportional-hazards model. Tumor cell heterogeneity was expected to increase the likelihood of the presence of a subclone with metastatic potential and resistant to standard treatment. However, increased intra-lesion heterogeneity in the LN metastases was strongly associated with better patient outcome even when controlled for clinical features such as tumor grade, molecular subtype, and nodal status. When the same analysis was performed on the primary tumors, no such association was observed, and there was also no association between heterogeneity of matched primary tumors and LN metastases. Patterns of association with heterogeneity were observed when modeling primary and disseminated sites independently (594 primary tumors and 213 LN metastases with survival information) and when modeling matched primary and LN metastases simultaneously (166 matched samples with survival information).

To determine whether heterogeneity in LN metastases was correlated with spatial separation or with mixing of phenotypes, the levels of heterogeneity were assessed within spatial communities of neighboring tumor cells (Jackson, H. W. 2020 Nature, 578:615). A mixing score was calculated for each tissue that reflects the average intra-community (i.e., local) heterogeneity. In this scoring system, the low values occur when a single tumor cell phenotype dominates the tissue or when there is no physical contact between different phenotypes, and high values occur when cells of many different phenotypes interact in every community. A strong correlation existed between LN metastasis heterogeneity and mixing of cellular phenotypes (i.e., a high score) and also a significant association of mixing with improved survival (Figure 6a, b). These findings indicate that better prognosis is associated with physically intermixed cell phenotypes instead of spatially separate but diverse subpopulations. Poor-prognosis LN metastases with low heterogeneity and low cellular mixing, produced by a single dominating tumor phenotype, were enriched for phenotypes considered more aggressive in primary tumors (TNBC and HER2+). Accordingly, community types composed of triple-negative or HER2+ cells, including the high-risk disseminated phenotype of epithelial cluster 29, showed low average levels of intra-community heterogeneity. Further, community types with high average heterogeneity were preferentially composed of hormone receptor-positive cells, which are generally considered less aggressive and more responsive to treatment (Figure 6c). Finally, spatial permutation analysis revealed that, despite phenotypic mixing in heterogeneous LNs, tumor cells in physical proximity were significantly more similar to each other than to distant cells in the vast majority of LN tissues (Figure 6c). In summary, heterogeneity and spatial mixing of cell phenotypes in LN metastases, but not of primary tumors, were associated with improved patient prognosis. The heterogeneous metastatic lesions showed local similarity in cellular phenotypes, which could be due to seeding by less advanced tumor cells that are more prone to differentiate.

### Features of the primary tumour are associated with prognostic disseminating cells

Correlations between disseminating cell phenotypes associated with poor prognosis and cellular phenotypes in the primary tumours assessed using the binary presence or absence measures of all tumour phenotypes identified. A correlation was observed between the good-prognosis, hormone receptor-positive disseminated cell phenotypes and the presence of luminal and hormone receptor-positive phenotypes in the primary tumour and a correlation between the dangerous p53+ triple-negative disseminated cell cluster and non-luminal cell clusters in the primary tumour tissue (Figure 7). Despite the frequent presence of non-luminal, triple-negative phenotypes in primary tumours of patients with dangerous disseminating cells, less than 25% of these patients were clinically categorized as TNBC, and the remaining tumours were from all molecular subtypes (Figure 4a). In these cases, the triple-negative cells likely constituted rare subpopulations of the primary tumours, which were not detected using standard histology.

Next, lasso-regularized logistic regression models identified primary tumour phenotypes predictive of the presence or absence of risk-associated disseminated cell phenotypes. Positive predictors in the primary tumour always included the same cellular phenotype as that of the disseminated cells in question (Figure 7), suggesting that the presence of these phenotypes in the primary tumour can be used as an indicator for risk-associated disseminated phenotypes. The two disseminated cell phenotypes with the greatest opposing impacts, epithelial cluster 9 (good-prognosis disseminating cell phenotype) and epithelial cluster 29 (poor-prognosis disseminating cell phenotype), were analysed for all 402 primary tumour samples of node-positive patients. These two phenotypes were rarely both present in the same tissue sample, whether primary tumour or LN metastasis (Figure 4a). For cluster 9, the majority of positive LNs originated from primary tumours in which this phenotype was also present (Figure 4a), although cells of cluster 9 were also often found in primary tumours for which the matched disseminated phenotypes did not include cells of this cluster (Figure 2d, Figure 4a). In node-positive patients with primary tumours containing cells that belong to cluster 29, the same phenotype was identified in most LN metastases (Figure 4a). Without considering information from a matched LN sample, node-positive patients with cells of cluster 29 in the primary tumour, and without cells of cluster 9, had poorer overall survival than those with primary tumours lacking cells belonging to this cluster (Figure 4b). This was the case excluding high-risk, clinically diagnosed TNBC samples (Figure 8), thereby demonstrating the independent prognostic power of this dissemination-prone phenotype. Interestingly, however, the small fraction of patients who had cells of cluster 29 in the primary tumour but who did not have LN metastases did not have poor prognosis (44 out of 256 patients without affected LNs (N0), Figure 4b).

### p53 and GATA3 expression in lymph node metastases is associated with patient survival

Multiplexed imaging allows for the identification of complex single-cell phenotypes in cancer tissues but the current clinical practice relies on standard single-stain immunohistochemistry. In order to simplify the identification of prognostic disseminated phenotypes to minimal relevant markers, associations between average marker expression levels in LN metastases and patient survival were examined using a lasso regularized Cox proportional-hazards model with the average disseminated cell expression levels of all markers, in order to identify those that stratify the patients according to overall survival. In accordance with the marker signatures of the prognostic disseminated phenotypes (Figure 3), the strongest opposing prognostic impact based on average marker expression was associated with p53 (**increased levels = bad prognosis**) and GATA3 (**increased levels = good prognosis**) (Figure 9a). Marker expression levels of p53 and GATA3 in the LN metastases yielded more prognostic information than any of the clinical patient classifications when modelled simultaneously (Figure 9b).

To demonstrate that p53 and GATA3 can be used as diagnostic markers with established clinical methods, slides were stained and single-cell marker expression levels quantified in different sections of the same LN metastasis cores using standard immunohistochemistry (Figure 10). QuPATH quantified the average tumour cell expression levels as well as fractions of highly expression tumour cells for p53, GATA3 and the clinically established markers (ER, PR and Ki-67). Modelling patient survival using a Cox proportional-hazards model revealed that with either measure (average expression or fraction of highly expressing cells) p53 and GATA3 were individually significantly associated with patient survival (Figure 9c and e). Furthermore, adding p53 and GATA3 to a model containing only the standard clinical markers (ER, PR and Ki-67) significantly improved the model (Figure 9d and f). Thereby, expression levels of p53 and GATA3 in LN metastases yield prognostic information not captured by clinically established markers, and can be measured using standard clinical methods.

Patient prognosis was observed to increase as GATA3 expression increased, or as less p53 was detected, however the survival of patients below or above particular defined thresholds could also provide distinct patient prognosis. Thresholds for either average biomarker expression levels across all tumour cells of a LN metastasis core, or the proportion of highly expressing cells could effectively discriminate groups with differing patient prognosis (Figure 11).

### Clinical implications

Survival analysis identified disseminating tumor cell phenotypes associated with distinct prognoses, potentially indicative of different metastatic potential or, alternatively, of different therapeutic responses. For instance, hormone receptor-positive disseminated cells, identified with GATA3 expression by IHC, were associated with good prognosis, and are potentially more sensitive to standard endocrine therapy than other phenotypes. Patients with LN metastases of such lower risk, hormone receptor-positive phenotypes may not need radiation, or aggressive chemotherapy with non-targeted anti-neoplastic drugs in addition to endocrine therapies, and could benefit from studies assessing risks of overtreatment. A non-luminal triple-negative disseminated phenotype characterized by high levels of p53 expression was associated with poor prognosis. These patients are unlikely to benefit from hormone-targeted therapy, and might be advised to enter rapid treatment with aggressive systemic chemotherapy, or combinations of different chemotherapy, perhaps additionally with radiation. Notably, this phenotype was observed in patients with primary tumors spanning all clinically assigned molecular subtypes, showing that this dangerous phenotype is not captured by conventional subtyping of the primary tumor. Risk-associated disseminated phenotypes provided prognostic information beyond that of nodal status alone. Thus, the phenotypes of metastatic cells, frequently distinct from those of the primary tumor, has a strong influence on disease outcome, indicating that the phenotypes of disseminated cells should be considered when treatment decisions are made.

### Example 2. Methods

### Clinical data

The patient samples and clinical information associated with the breast cancer cohort described in this study were obtained from University Hospital Zurich. Pathologists designed and constructed three tissue microarrays (TMAs) (ZTMA21, ZTMA25 and ZTMA26) of core biopsies from different tissue types of breast cancer patients with a focus on primary tumours and LN metastases, taken at diagnosis and before treatment. The TMAs contain a single 0.6-mm diameter core per patient and available tissue type. Images were obtained of 771 primary tumours, 271 LN metastases, 49 tumour recurrences, 41 distant metastases, and 37 healthy breast tissue samples from 890 patients, resulting in 1245 images. For 263 patients more than one tissue type was available and for 212 patients both a primary tumour and a LN metastasis core were available. For analysis, a minimum threshold of 100 tumour cells was applied per image, in order to exclude cores that may have missed the tumour. As a result, the number of patients used for paired primary tumour and LN metastasis analysis was reduced to 203. This project was approved by the local Commission of Ethics (BASEC-PB-2019-111).

### Multiplex sample preparation

The antibody panel was designed to focus on breast cancer-specific epitopes but also to distinguish epithelial, mesenchymal, endothelial, and the main immune cell types (Tab. 1). Slides were stained as previously described (Shapiro, D. 2017 Nat. Methods: 14:873).

### Imaging mass cytometry

Multiplexed images of the TMA cores were acquired using a Hyperion Imaging System (Fluidigm). A square area around each core of a TMA was acquired at 400 Hz, and the raw data were preprocessed using commercial software (Fluidigm). The cores within a TMA were acquired in a randomized order, and the three TMAs were acquired in one continuous run in order of increasing ZTMA number. In cases of unexpectedly interrupted acquisitions, the remaining part of the core was acquired as a separate image, resulting in few patients with the same core split into two images. Signal spillover between channels was compensated on the single-cell level using the CATALYST R package (v.1.12.1).

### Data processing

The commercial image format was converted to OME-TIFF files, then segmented into single-cells using a combination of ilastik (v.1.3.3, Berg, S. 2019 Nat. Methods 16:e2005970) and CellProfiler (v.3.1.8, McQuin, C. PloS Biol. 2018 16:e2005970), following the imaginf mass cytmoetry Segmentation Pipeline (v0.9). High-dimensional mean marker expressions and spatial single-cell features were extracted using CellProfiler. Cells on the edges of the cores were marked as special cases for spatial analyses, and a circular area of each core was recorded for downstream density calculations. Even with high-quality segmentation, single-cells extracted from images of tissue sections represent tissue slices with potentially overlapping cell fragments. Therefore, the extracted single-cell marker expression can include some information of neighboring cells, especially in densely packed tissues. Cellular neighborhoods were extracted by expanding the circumference of each cell by 4 pixels (4 µm) and recording the overlapping neighbor cell IDs using CellProfiler. The pixel values in the area corresponding to each cell were averaged into mean single-cell marker expressions. The single-cell expression data were normalized between 0 and 1 for each marker using the 99th percentile normalization to account for outliers. All downstream analyses were conducted in R (v.3.6.3). A size threshold was applied to mark extremely small or extremely large cells as potential mis-segmentations and remove them from analyses. Only cores containing a minimum of 100 tumour cells were considered for analysis, in order to exclude potentially misleading cores, which may have missed the tumour bulk. Whenever binary presence or absence of a cell type in a tissue is reported, the threshold for presence was 5 cells rather than 1, in order to increase robustness of the analysis. The "predominant" cell type of a tissue was defined as the most abundant phenotype identified in the core.

### Clustering

To identify single-cell phenotypes, epithelial cells were subjected to unsupervised clustering separately from non-epithelial cells. The R package mclust (v.5.4.6) applied a Gaussian-mixture model to the mean single-cell expressions of panCK, in order to separate panCK⁺ from panCK⁻ cells. However, certain tumour phenotypes, especially non-luminal triple-negative cells, can have very low expression levels of panCK and may be mistaken for non-epithelial cells by a Gaussian-mixture model. Therefore, an unsupervised clustering step, using the RPhenoGraphimplementation from cytofkit (v.1.10.0, Levine, J. H. Cell 2015 162:184) was used, based on all markers and with a nearest neighbour parameter of 50. This granular clustering step split the single cells into 130 clusters. Each cluster was assigned to the epithelial or non-epithelial subgroup based on the majority assignment of cells by the Gaussian-mixture model, refined when neccesary. The epithelial subclustering was performed using the RPhenoGraph implementation of the cytofkit R package with k = 100 for epithelial and k = 50 for non-epithelial cells. The higher nearest neighbour parameter for epithelial cell clustering was chosen to balance for the higher number of epithelial markers and large inter-patient variability among tumour cells, which would result in exploding numbers of clusters at low nearest neighbour parameters. Epithelial cell clustering resulted in 59 clusters. The z-scored mean marker levels of the clusters are displayed in Figure 1c.

### Differential abundance of cell types between matched primary tumours and LN metastases

Differential abundance testing was conducted using R functions of the edgeR package (v.3.28.0), inspired by the workflow described and implemented in the R package diffcyt (v.1.6.0). In addition to the tissue type of each sample, the model was provided with the patient IDs, thereby accounting for the paired design of the primary tumours and LN metastases. Normalization factors, calculated via the trimmed mean of M-values method, were included to adjust for composition effects. This analysis was conducted separately on epithelial and non-epithelial cells. A significance threshold of p < 0.01 was used to identify the differentially abundant cell types.

### Survival analysis

The R package survival (v.3.1-12) was used to calculate Kaplan-Meier survival curves and Cox proportional-hazards models. The number of samples used for survival analysis slightly deviated from the described total number of samples because survival information was not available for every patient in the cohort. Occasionally, other missing clinical information also led to the exclusion of some patients from analysis. When the number of predictors to be assessed was too high compared to the number of samples to use a regular Cox proportional-hazards model (e.g., 59 epithelial phenotypes, 213 LN metastasis samples), lasso-regularized Cox proportional-hazards models were calculated using the cv.glmnet function of the R package glmnet (v.3.0-2) with the family "cox" option (Friedman J, J. Stat. Software 2010 33(1):1; Simon N, J. Stat. Software 2011 39(5):1). To increase robustness, the cross validation was run 500 times, and the mean error curves were averaged before choosing the optimal lambda value. The cell-type covariates were based on binary presence or absence values and therefore treated like other categorical variables and not standardized. Hazard ratios of the active covariates of the model at the minimum CV-error are displayed in the figures. The hazard ratios of the lasso selected predictors are displayed without confidence intervals, as p-values and confidence intervals of individual coefficients are invalid after feature selection.

### Predicting prognostic disseminated cell types based on the primary tumour

To identify phenotypes in the primary tumour that are predictive of the presence of a disseminated cell type of interest, logistic regression models were applied to predict the binomial presence or absence of the phenotype of interest among the cells in the LN metastasis. Lasso-regularization was used to select the stratifying covariates. The cv.glmnet function of the R package glmnet (v.3.0-2) was used with family "binomial" and 10-fold cross validation. To increase robustness, the cross validation was run 500 times, and the mean error curves were averaged before choosing the optimal lambda value. The cell type predictors were based on binary presence/absence values and therefore treated like other categorical variables and not standardized. The active predictors of the model at the minimum CV-error are displayed in the figures.

### IHC quantification:

Pixel or single-cell level example images shown in the figures were generated using the cytomapper R package. IHC stains on the TMAs were performed at the University Hospital Zurich, with anti-GATA3 and p53 anti bodies and a standard horseradish peroxidase enzymatic detection system, and haematoxylin counterstaining. Using QuPATH, each core in the scanned TMA images was segmented into single-cells, which were classified into tumour and non-tumour cells based on manual training. Average expression levels of tumour cells as well as the fraction of highly expressing tumour cells, defined as 10 fold increased staining intensity over background cell staining obtained from healthy liver samples, were extracted for each core.

**Table . 1**

| Metal Tag | Target | Antibody Clone | Company | Catalogue Number |
|---|---|---|---|---|
| In113 | Histone H3 | D1H2 | Cell Signaling | 4499 |
| La139 | H3 (Lys28) trimethylate | C36B11 | Cell Signaling | 9733 |
| Pr141 | Cytokeratin 5 | EP1601Y | Abcam | ab214586 |
| Nd142 | Fibronectin | 10/Fibronectin | Becton Dickinson | 610078 |
| Nd143 | HLA-DR | TAL 1B5 | Abcam | ab20181 |
| Nd144 | Cytokeratin 8/18 | C51 | Cell Signaling | 4546 |
| Nd145 | CD15 | HI98 | Biolegend | 301902 |
| Nd146 | CD68 | KP1 | E-Bioscience | 14-0688-82 |
| Sm147 | Keratin 14 (KRT14) | polyclonal_PA5-16722 | Thermo Fischer | PA5-16722 |
| Nd148 | SMA | 1A4 | Abcam | 7817 |
| Sm149 | Vimentin | D21H3 | Cell Signaling | 5741 |
| Nd150 | c-Myc | 9E10 | Biolegend | 626802 |
| Eu151 | c-erbB-2 - Her2 | 3B5 | Becton Dickinson | 554299 |
| Sm152 | CD3 | polyclonal_A0452 | Dako | A0542 |
| Eu153 | p-Histone H3 | HTA28 | Biolegend | 641002 |
| Sm154 | CD11c | EP1347Y | Abcam | ab216655 |
| Gd155 | Bcl-2 | 100 | Biolegend | 658702 |
| Gd156 | Estrogen Receptor Alpha | EP1 | Epitomics | IR084 |
| Gd156 | Estrogen Receptor alpha | EPR4097 | Abcam | ab108398 |
| Gd156 | Estrogen Receptor Alpha | SP1 | Abcam | ab187260 |
| Gd158 | Progesterone Receptor A/B | EP2 | Epitomics | AC-0028EU |
| Gd158 | Progesterone Receptor A/B | SP2 | Abcam | ab239793 |
| Tb159 | p53 | 7F5 | Cell Signaling | 2527 |
| Gd160 | CD44 | polyclonal_CD44 | R&D Systems | AF3660 |
| Dy161 | Androgen Receptor AR | D6F11 | Cell Signaling | 5153 |
| Dy162 | CD45RO | UCHL1 | Biolegend | 304202 |
| Dy162 | CD45RA | HI100 | Biolegend | 304120 |
| Dy162 | CD45 | 2B11 | E-Biosciences | 14-9457-82 |
| Dy163 | GATA3 | L50-823 | Bectin Dickinson | 558686 |
| Dy164 | CD20 | L26 | E-Biosciences | 14-0202-82 |
| Ho165 | CD8a | C8/144B | E-Biosciences | 14-0085-82 |
| Er166 | Carbonic Anhydrase IX | polyclonal CA9 AF2188 | R&D Systems | AF2188 |
| Er167 | E-Cadherin / P-Cadherin | 36/E-Cadherin | Bectin Dickinson | 610182 |
| Er168 | Ki-67 | B56 | BD Biosciences | 556003 |
| Tm169 | EGFR | D38B1 | Cell Signaling | 4267 |
| Er170 | p-S6 | D57.2.2E | Cell Signaling | 4858 |
| Yb171 | CD4 | Polyclonal_CD4_AF-379 | R&D Systems | AF-379 |
| Yb172 | vWF | poly vwf | Millipore | AB7356 |
| Yb172 | CD31 | EPR3094 | Abcam | ab207090 |
| Yb173 | p-mTOR | 49F9 | Cell Signaling | 2976 |
| Yb174 | Cytokeratin 7 | RCK105 | Abcam | ab9021 |
| Lu175 | pan Cytokeratin | AE3 | Millipore | MAB1611 |
| Lu175 | pan Cytokeratin | AE1 | Millipore | MAB1612 |
| Yb176 | Cleaved Caspase3 | C92-605 | Bectin Dickinson | 559565 |
| Yb176 | cleaved PARP | F21-852 | BD Biosciences | 552596 |

## Claims

1. A method of determining a prognosis of a breast cancer patient comprising the following steps:
a. contacting a lymph node metastasis sample obtained from the patient with a first molecular probe specific for GATA 3 conjugated to a detectable label, and a second molecular probe, conjugated to a detectable label, and specific for p53;
b. determining an expression level of GATA3 and an expression level of p53;
c. assigning to the patient a good prognosis if the patient sample is **characterised by** a GATA3 expression level greater than or equal to (≥) a GATA3 threshold, and/or a p53 expression level below (<) a p53 threshold, or
assigning to the patient a poor prognosis if the patient sample is **characterised by** a p53 expression level ≥ the p53 threshold, and/or a GATA3 expression level < the GATA3 threshold;
and
wherein the biomarker expression level is determined at the level of the whole sample, or a portion of the sample comprising a plurality of cells, and wherein
- the GATA3 threshold is 100 times ≥ a negative control expression level; and
- the p53 is threshold is ≥ 8 times a negative control expression level;
and wherein the negative control expression level is the biomarker expression level determined for a negative control sample;
or
wherein the biomarker expression level is determined at the level of a single cell for each of a plurality of individual cells present in the sample, wherein
- the GATA3 threshold is ≥ 20% GATA3 high cells; and
- the p53 threshold is ≥ 6% p53 high cells,
and wherein a cell is classified as high for biomarker expression if the biomarker expression level of the cell is ≥ 10 times the biomarker expression level of a cell in a negative control sample.

2. The method according to claim 1, wherein good prognosis indicates at least about 60% probability of survival at 300 months, and/or poor prognosis indicates less than about 50% probability of survival at 100 months.

3. The method according to claim 1 or 2, wherein good prognosis indicates about 60% probability of survival at 300 months, and/or poor prognosis indicates about 10% probability of survival at 100 months.

4. The method according to any one of the claims 1 to 3, wherein the molecular probe is an antibody, antibody-like molecule and/or nucleic acid probe, particularly wherein the molecular probe is an antibody.

5. The method according to any one of the claims 1 to 4, wherein the method of obtaining information about the expression level of the biomarkers GATA3 and/or p53 comprises obtaining an image of the ex vivo lymph node metastasis sample.

6. The method according to any one of the claims 1 to 5, wherein the sample is a monolayer of adherent ex vivo lymph node metastasis cells or ex vivo lymph node metastasis cells otherwise immobilised on a solid surface.

7. The method according to any one of the claims 1 to 6, wherein the sample is a tissue section of an ex vivo lymph node metastasis sample.

8. The method according to any one of the claims 1 to 7, wherein the method of obtaining information about the expression level of the biomarkers GATA3 and/or p53 is
a. imaging mass cytometry at a subcellular resolution, particularly at a resolution of ≤5µm, or even ≤1µm, or
b. immunohistochemistry.

9. Use of a kit comprising a molecular probe specific for biomarkers p53, and a molecular probe specific for GATA3, and each molecular probe bearing a detectable label, for carrying out the method according to any one of the claims 1 to 8.

10. Use of a system for carrying out the method according to any one of the claims 1 to 8, wherein the system comprises a molecular probe bearing a detectable label with specificity for p53, and a molecular probe bearing a detectable label with specificity for GATA3, and a device for analysing the amount of detectable label in a lymph node metastasis sample

## Patentansprüche

1. Verfahren zur Ermittlung einer Prognose für einen Brustkrebspatienten, das die folgenden Schritte umfasst:
a. Inkontaktbringen einer aus dem Patienten erhaltenen Lymphknotenmetastasen-Probe mit einer für GATA3 spezifischen ersten molekularen Sonde, die mit einer nachweisbaren Markierung konjugiert ist, und einer zweiten molekularen Sonde, die mit einer nachweisbaren Markierung konjugiert ist und für p53 spezifisch ist;
b. Ermitteln eines Expressionsniveaus von GATA3 und eines Expressionsniveaus von p53;
c. Zuweisen einer guten Prognose für den Patienten, wenn die Patientenprobe durch ein GATA3-Expressionsniveau, das größer als oder gleich (≥) einem GATA3-Schwellenwert ist, und/oder ein p53-Expressionsniveau, das kleiner als (<) ein p53-Schwellenwert ist, gekennzeichnet ist, oder
Zuweisen einer schlechten Prognose für den Patienten, wenn die Patientenprobe durch ein p53-Expressionsniveau ≥ dem p53-Schwellenwert und/oder ein GATA3-Expressionsniveau < der GATA3-Schwellenwert gekennzeichnet ist;
und
wobei das Biomarker-Expressionsniveau auf der Ebene der gesamten Probe oder eines Abschnitts der Probe, der eine Vielzahl von Zellen umfasst, ermittelt wird, und wobei
- der GATA3-Schwellenwert ≥ dem 100-Fachen eines Negativkontrolle-Expressionsniveaus ist; und
- der p53-Schwellenwert ≥ dem 8-Fachen eines Negativkontrolle-Expressionsniveaus ist;
und wobei das Negativkontrolle-Expressionsniveau das für eine Negativkontrollprobe ermittelte Biomarker-Expressionsniveau ist;
oder
wobei das Biomarker-Expressionsniveau auf der Ebene einer einzelnen Zelle für jede von einer Vielzahl von Einzelzellen, die in der Probe vorhanden sind, ermittelt wird, wobei
- der GATA3-Schwellenwert ≥ 20 % GATA3-starker Zellen ist; und
- der p53-Schwellenwert ≥ 6 % p53-starker Zellen ist,
und wobei eine Zelle als stark hinsichtlich der Biomarker-Expression eingestuft wird, wenn das Biomarker-Expressionsniveau der Zelle ≥ dem 10-Fachen des Biomarker-Expressionsniveaus einer Zelle in einer Negativkontrollprobe ist.

2. Verfahren nach Anspruch 1, wobei eine gute Prognose eine Überlebenswahrscheinlichkeit von mindestens etwa 60 % nach 300 Monaten angibt, und/oder eine schlechte Prognose eine Überlebenswahrscheinlichkeit von weniger als etwa 50 % nach 100 Monaten angibt.

3. Verfahren nach Anspruch 1 oder 2, wobei eine gute Prognose eine Überlebenswahrscheinlichkeit von etwa 60 % nach 300 Monaten angibt, und/oder eine schlechte Prognose eine Überlebenswahrscheinlichkeit von etwa 10 % nach 100 Monaten angibt.

4. Verfahren nach einem beliebigen der Ansprüche 1 bis 3, wobei die molekulare Sonde ein Antikörper, ein antikörperähnliches Molekül und/oder eine Nucleinsäuresonde ist, wobei die molekulare Sonde insbesondere ein Antikörper ist.

5. Verfahren nach einem beliebigen der Ansprüche 1 bis 4, wobei das Verfahren zum Erhalten von Informationen über das Expressionsniveau der Biomarker GATA3 und/oder p53 ein Erhalten eines Bilds der Ex-vivo-Lymphknotenmetastasen-Probe umfasst.

6. Verfahren nach einem beliebigen der Ansprüche 1 bis 5, wobei es sich bei der Probe um eine Monoschicht von adhärenten Ex-vivo-Lymphknotenmetastasen-Zellen oder Ex-vivo-Lymphknotenmetastasen-Zellen, die anderweitig auf einer festen Oberfläche immobilisiert sind, handelt.

7. Verfahren nach einem beliebigen der Ansprüche 1 bis 6, wobei die Probe ein Gewebeschnitt einer Ex-vivo-Lymphknotenmetastasen-Probe ist.

8. Verfahren nach einem beliebigen der Ansprüche 1 bis 7, wobei das Verfahren zum Erhalten von Informationen über das Expressionsniveau der Biomarker GATA3 und/oder p53 Folgendes ist
a. bildgebende Massenzytometrie mit subzellulärer Auflösung, insbesondere mit einer Auflösung von ≤5 µm oder sogar ≤1 µm, oder
b. Immunhistochemie.

9. Verwendung eines Kits zur Durchführung des Verfahrens nach einem beliebigen der Ansprüche 1 bis 8, das eine für Biomarker p53 spezifische molekulare Sonde und eine für GATA3 spezifische molekulare Sonde umfasst, und wobei jede molekulare Sonde eine nachweisbare Markierung trägt.

10. Verwendung eines Systems zur Durchführung des Verfahrens nach einem beliebigen der Ansprüche 1 bis 8, wobei das System eine molekulare Sonde, die eine nachweisbare Markierung mit Spezifität für p53 trägt, und eine molekulare Sonde, die eine nachweisbare Markierung mit Spezifität für GATA3 trägt, und eine Vorrichtung zum Analysieren der Menge an nachweisbarer Markierung in einer Lymphknotenmetastasen-Probe umfasst.

## Revendications

1. Procédé de détermination d'un pronostic d'une patiente atteinte de cancer du sein comprenant les étapes suivantes :
a. mettre en contact un échantillon de métastase de ganglion lymphatique obtenu auprès de la patiente avec une première sonde moléculaire spécifique à GATA 3 conjuguée à un marqueur détectable, et une seconde sonde moléculaire, conjuguée à un marqueur détectable, et spécifique à p53 ;
b. déterminer un niveau d'expression de GATA3 et un niveau d'expression de p53 ;
c. attribuer à la patiente un bon pronostic si l'échantillon de la patiente est **caractérisé par** un niveau d'expression de GATA3 supérieur ou égal à (≥) un seuil de GATA3, et/ou un niveau d'expression de p53 inférieur à (<) un seuil de p53, ou attribuer à la patiente un mauvais pronostic si l'échantillon de la patiente est **caractérisé par** un niveau d'expression de p53 ≥ au seuil de p53, et/ou un niveau d'expression de GATA3 < au seuil de GATA3 ;
et
dans lequel le niveau d'expression de biomarqueur est déterminé au niveau de l'échantillon entier, ou d'une portion de l'échantillon comprenant une pluralité de cellules, et dans lequel
- le seuil de GATA3 est 100 fois ≥ à un niveau d'expression de témoin négatif ; et
- le seuil de p53 est ≥ à 8 fois un niveau d'expression de témoin négatif ;
et dans lequel le niveau d'expression de témoin négatif est le niveau d'expression de biomarqueur déterminé pour un échantillon témoin négatif ;
ou
dans lequel le niveau d'expression de biomarqueur est déterminé au niveau d'une seule cellule pour chacune d'une pluralité de cellules individuelles présentes dans l'échantillon, dans lequel
- le seuil de GATA3 est ≥ à 20 % de cellules élevées en GATA3 ; et
- le seuil de p53 est ≥ à 6 % de cellules élevées en p53,
et dans lequel une cellule est classifiée comme élevée pour l'expression de biomarqueur si le niveau d'expression de biomarqueur de la cellule est ≥ à 10 fois le niveau d'expression de biomarqueur d'une cellule dans un échantillon témoin négatif.

2. Procédé selon la revendication 1, dans lequel un bon pronostic indique au moins environ 60 % de probabilité de survie à 300 mois, et/ou un mauvais pronostic indique moins d'environ 50 % de probabilité de survie à 100 mois.

3. Procédé selon la revendication 1 ou 2, dans lequel un bon pronostic indique environ 60 % de probabilité de survie à 300 mois, et/ou un mauvais pronostic indique environ 10 % de probabilité de survie à 100 mois.

4. Procédé selon l'une quelconque des revendications 1 à 3, dans lequel la sonde moléculaire est un anticorps, une molécule de type anticorps et/ou une sonde d'acide nucléique, notamment dans lequel la sonde moléculaire est un anticorps.

5. Procédé selon l'une quelconque des revendications 1 à 4, dans lequel le procédé d'obtention d'informations concernant le niveau d'expression des biomarqueurs GATA3 et/ou p53 comprend l'obtention d'une image de l'échantillon de métastase de ganglion lymphatique ex vivo.

6. Procédé selon l'une quelconque des revendications 1 à 5, dans lequel l'échantillon est une monocouche de cellules de métastase de ganglion lymphatique ex vivo adhérentes ou des cellules de métastase de ganglion lymphatique ex vivo autrement immobilisées sur une surface solide.

7. Procédé selon l'une quelconque des revendications 1 à 6, dans lequel l'échantillon est une coupe de tissu d'un échantillon de métastase de ganglion lymphatique ex vivo.

8. Procédé selon l'une quelconque des revendications 1 à 7, dans lequel le procédé d'obtention d'informations concernant le niveau d'expression des biomarqueurs GATA3 et/ou p53 est
a. l'imagerie de cytométrie de masse à une résolution subcellulaire, notamment à une résolution de ≤5 µm, ou même ≤1 µm, ou
b. l'immunohistochimie.

9. Utilisation d'un kit comprenant une sonde moléculaire spécifique à des biomarqueurs p53, et une sonde moléculaire spécifique à GATA3, et chaque sonde moléculaire portant un marqueur détectable, pour réaliser le procédé selon l'une quelconque des revendications 1 à 8.

10. Utilisation d'un système pour réaliser le procédé selon l'une quelconque des revendications 1 à 8, dans laquelle le système comprend une sonde moléculaire portant un marqueur détectable avec une spécificité à p53, et une sonde moléculaire portant un marqueur détectable avec une spécificité à GATA3, et un dispositif pour analyser la quantité de marqueur détectable dans un échantillon de métastase de ganglion lymphatique.
